(19) Europäisches Patentamt
European Patent Office
Office européen des brevets

(11) **EP 2 539 323 B3**

(12) **NEUE EUROPÄISCHE PATENTSCHRIFT**
Nach dem Beschränkungsverfahren (B3-1)


(45) Hinweis auf die Patenterteilung:
**03.12.2014 Patentblatt 2014/49**

(45) Veröffentlichungstag und Bekanntmachung des Hinweises auf die Entscheidung über den Antrag auf Beschränkung:
**B3-1 04.10.2017 Patentblatt 2017/40**

(21) Anmeldenummer: **11705858.6**

(22) Anmeldetag: **21.02.2011**

(51) Int Cl.:
***C07D 211/62*** (2006.01) ***C07D 213/81*** (2006.01)
***C07D 233/90*** (2006.01) ***C07D 239/28*** (2006.01)
***C07D 261/18*** (2006.01) ***C07D 271/10*** (2006.01)
***C07D 239/557*** (2006.01) ***C07D 207/277*** (2006.01)
***C07C 237/24*** (2006.01) ***A61K 31/165*** (2006.01)
***A61K 31/44*** (2006.01) ***A61K 31/505*** (2006.01)
***A61K 31/506*** (2006.01) ***C07D 401/12*** (2006.01)
***C07D 405/12*** (2006.01)

(86) Internationale Anmeldenummer:
**PCT/EP2011/052512**

(87) Internationale Veröffentlichungsnummer:
**WO 2011/104203 (01.09.2011 Gazette 2011/35)**


(54) **Verbindungen als Bradykinin-B1-Antagonisten**

Compounds as bradykinin B1 antagonists

Composés comme antagonistes de la bradykinine B1


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **23.02.2010 PCT/EP2010/052232**

(43) Veröffentlichungstag der Anmeldung:
**02.01.2013 Patentblatt 2013/01**



(73) Patentinhaber: **Boehringer Ingelheim International GmbH**
**55216 Ingelheim am Rhein (DE)**

(72) Erfinder:
- **HAUEL, Norbert**
  **55216 Ingelheim am Rhein (DE)**
- **CECI, Angelo**
  **55216 Ingelheim am Rhein (DE)**
- **DOODS, Henri**
  **55216 Ingelheim am Rhein (DE)**
- **KONETZKI, Ingo**
  **55216 Ingelheim am Rhein (DE)**
- **MACK, Juergen**
  **55216 Ingelheim am Rhein (DE)**
- **PRIEPKE, Henning**
  **55216 Ingelheim am Rhein (DE)**
- **SCHULER-METZ, Annette**
  **55216 Ingelheim am Rhein (DE)**
- **WALTER, Rainer**
  **55216 Ingelheim am Rhein (DE)**
- **WIEDENMAYER, Dieter**
  **55216 Ingelheim am Rhein (DE)**

(74) Vertreter: **Simon, Elke Anna Maria et al**
**Boehringer Ingelheim GmbH**
**Binger Strasse 173**
**55216 Ingelheim am Rhein (DE)**



(56) Entgegenhaltungen:
**WO-A1-03/066577** **WO-A1-2005/016886**
**WO-A2-2004/019868** **WO-A2-2005/085198**





Printed by Jouve, 75001 PARIS (FR)

## Beschreibung

Gegenstand der vorliegenden Erfindung sind die Verbindungen der allgemeinen Formel I

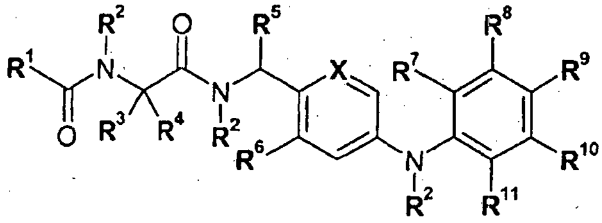

, (I)

in der **n, $R^1$, $R^2$, $R^3$, $R^4$, $R^5$, $R^6$, $R^7$, $R^8$, $R^9$, $R^{10}$, $R^{11}$** und **X** wie nachstehend beschrieben definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

## HINTERGRUND DER ERFINDUNG

### TECHNISCHES GEBIET

Die vorliegende Erfindung betrifft 3-Oxo-pyridazin-Verbindungen und deren Verwendung als B1-Rezeptor-Antagonisten, pharmazeutische Zusammensetzungen enthaltend diese Verbindungen sowie Methoden zur Verwendung derselben zur Vorbeugung oder Behandlung von akuten Schmerzen, Eingeweideschmerzen, neuropathischen Schmerzen, entzündlichen und Schmerzrezeptor-vermittelten Schmerzen, Tumorschmerzen sowie Kopfschrnerzen.

### STAND DER TECHNIK

In der internationalen Patentanmeldung PCT/EP2010/052232 bzw. in deren zugrunde liegender Prioritätsanmeldung werden bereits Verbindungen mit B1-antagonistischer Wirkung beschrieben. B1-Antagonisten der Strukturklasse Aminocyclopropancarboxamide werden in WO 2005/085198, der Strukturklasse N-Biphenylmethyl-aminocycloalkancarboxamide in WO 2003/066577, der Strukturklasse N-Biarylmethyl-aminocycloalkancarboxamide in WO 2004/019868, der Strukturklasse Sulfonyl-substituierte N-(Biarylmethyl)-aminocyclopropancarboxamide in WO 2005/016886.

Eine Aufgabe der vorliegenden Erfindung bestand darin, neue Verbindungen zur Verfügung zu stellen, die sich insbesondere als pharmakologische Wirkstoffe in Arzneimitteln eignen, die zur Behandlung von Krankheiten verwendet werden können, welche zumindest teilweise durch den B1-Rezeptor vermittelt werden.

Ein wesentliches strukturelles Merkmal der neuen Verbindungen ist die 6-Oxo-1,6-dihydro-pyridazin-4-carbonsäureamid-Gruppe, die im tautomeren. Gleichgewicht mit der 6-Hydroxy-pyridazin-4-carbonsäureamid-gruppe vorliegt:

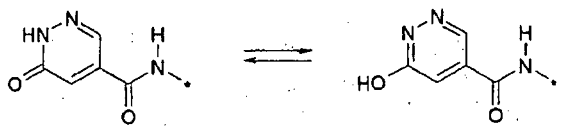

Gegenüber den Verbindungen aus dem Stand der Technik zeichnen sich die neuen Substanzen dadurch aus, dass sie eine starke B1-Rezeptor blockierende Wirkung zeigen und gleichzeitig eine verbesserte metabolische Stabilität aufweisen.

## DETAILLIERTE BESCHREIBUNG DER ERFINDUNG

In der obigen allgemeinen Formel **I** bedeuten in einer Ausführungsform <u>**1**</u>
**$R^1$** die Gruppe

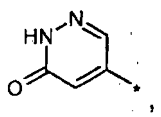

**$R^2$** H oder $CH_3$,
**$R^3$** und **$R^4$** zusammen mit dem Kohlenstoffatom, an dem sie gebunden sind, eine $C_{3-6}$-Cycloalkylengruppe, in der eine $-CH_2$-Einheit durch ein Sauerstoffatom ersetzt sein kann,
**$R^5$** H oder $CH_3$,
**$R^6$** H, F, Cl oder Methyl,
**$R^7$** H, F, Cl, Br, -CN, $C_{1-4}$-Alkyl, $CF_3$, $CHF_2$,
**$R^8$** H,
**$R^9$** F, Cl, Br, $C_{1-4}$-Alkyl, -O-$C_{1-4}$-Alkyl, -S-$C_{1-4}$-Alkyl,
**$R^{10}$** H,
**$R^{11}$** F, Cl, Br, -CN, $C_{1-4}$-Alkyl, $CF_3$, $CHF_2$, und
**X** CH oder N,
deren Enantiomere, deren Diastereomere, deren Gemische und deren Salze, insbesondere deren physiologisch verträgliche Salze mit organischen oder anorganischen Säuren oder Basen.

Eine Ausführungsform **2** der vorliegenden Erfindung besteht in den Verbindungen der allgemeinen Formel **I**, in denen **n, $R^1$ $R^3$, $R^4$, $R^5$, $R^6$, $R^7$, $R^8$, $R^9$, $R^{10}$, $R^{11}$** und X wie voranstehend in Ausführungsform 1 beschrieben definiert sind und
**$R^2$** H bedeutet,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **I** seien beispielsweise folgende genannt:

| Nr. | Struktur |
|---|---|
| (1) | 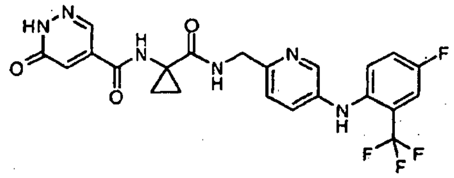 |
| (2) | 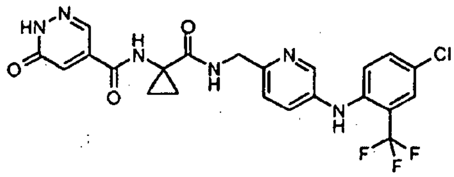 |
| (3) | 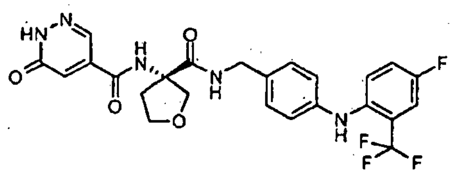 |
| (4) | 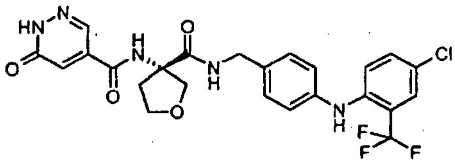 |

| Nr. | Struktur |
|---|---|
| (5) | 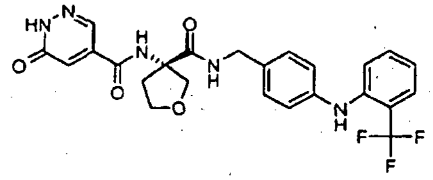 |
| (6) | 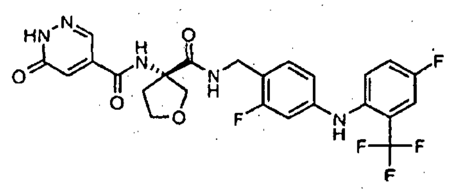 |
| (7) | 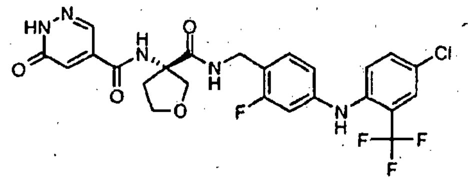 |
| (8) | 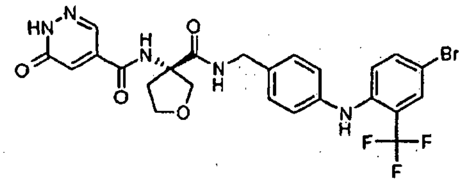 |
| (9) | 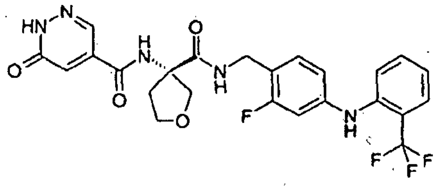 |

deren Enantiomere, deren Diastereomere, deren Gemische und deren Salze, insbesondere deren physiologisch verträgliche Salze mit organischen oder anorganischen Säuren oder Basen.

## VERWENDETE BEGRIFFE UND DEFINITIONEN

Soweit nicht anders angegeben, sind alle Substituenten voneinander unabhängig. Sollten an einer Gruppe z.B. mehrere $C_{1-4}$-Alkylgruppen als Substituenten sein, so könnte im Fall von drei Substituenten $C_{1-4}$-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "$C_{1-4}$-Alkyl" (auch soweit sie Bestandteil anderer Reste sind) werden Alkylgruppen mit 1 bis 4 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl und *tert*-Butyl. Gegebenenfalls werden für die vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl.

Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "$C_{3-6}$-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl.

Verbindungen derallgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oderp-Toluolsulfonsäure in Frage, als organische Säuren kommen beispielsweise Äpfelsäure, Bemsteinsäure, Essigsäure, Fumarsäure, Maleinsäure, Mandelsäure, Milchsäure, Weinsäure oder Zitronensäure in Betracht.

Weiterhin lassen sich die Verbindungen der allgemeinen Formel **I,** sofern sie geeignete Carbonsäurefunktionen enthalten, insbesondere für pharmazeutische Anwendungen in ihre physiologisch verträglichen Salze mit anorganischen öder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Verbindungen mit einer Kohlenstoff-Doppelbindung können sowohl in der E- als auch Z-Form vorliegen.

Falls eine Verbindung in verschiedenen tautomeren Formen vorliegen kann, so ist die dargestellte Verbindung nicht auf eine tautomere Form beschränkt, sondern umfasst alle tautomeren Formen. Dies gilt insbesondere auch bei stickstoffhaltigen Heteroarylen:

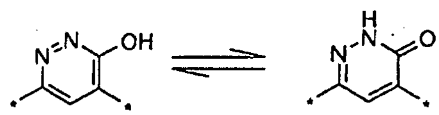

**HERSTELLVERFAHREN**

Erfindungsgemäß erhält man die Verbindungen der allgemeinen Formel **I** nach an sich bekannten Verfahren, beispielsweise nach folgenden Verfahren:

(A) Amid-Kupplung:

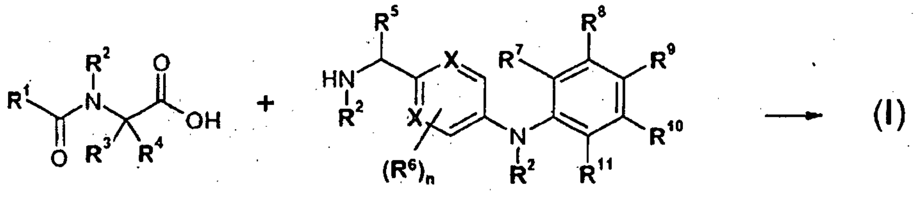

**(II)** **(III)**

Die dargestellte Verknüpfung von Carbonsäuren der allgemeinen Formel **II**, in der alle Reste wie vorstehend erwähnt definiert sind, mit Aminen der allgemeinen Formel **III,** in der alle Reste wie vorstehend erwähnt definiert sind, unter. Bildung von Carbonsäureamiden der allgemeinen Formel **I,** in der alle Reste wie vorstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B.

Dicyclohexylcarbodiimid (DCC), Diisopropylcarbodiimid (DIC) oder Ethyl-(3-dimethylamino-propyl)-carbodiimid, *O*-(1*H*-Benzotriazol-1-yl)-*N,N-N',N'*-tetramethyluronium-hexafluorphosphat (HBTU) oder-tetrafluorborat (TBTU) oder 1*H*-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran (THF), Acetonitril, Dimethylformamid (DMF), Dimethylacetamid (DMA), *N*-Methylpyrrolidon (NMP) oder Gemischen aus diesen durchgeführt. Sofern erforderlich wird zusätzlich eine Hilfsbase wie beispielsweise Diisopropylethylamin (DIPEA, Hünig-Base) eingesetzt.

(B) Amid-Kupplung:

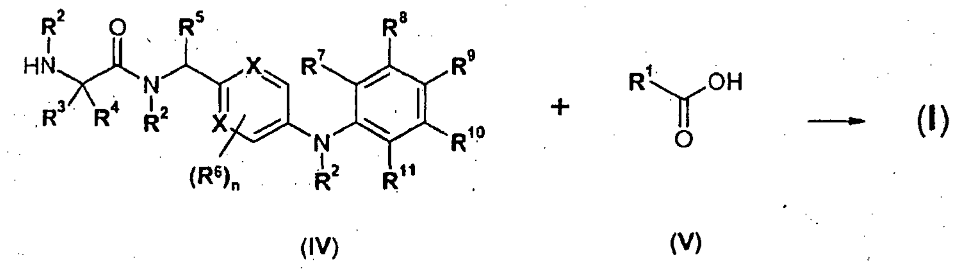

(IV) (V)

Eine alternative Methode zur Darstellung von Verbindungen der allgemeinen Formel **I** besteht in der Verknüpfung von Carbonsäuren der allgemeinen Formel V, in der alle Reste wie vorstehend erwähnt definiert sind, mit Aminen der allgemeinen Formel **IV,** in der alle Reste wie vorstehend erwähnt definiert sind.

Die Verbindungen der allgemeinen Formel **V** sind entweder käuflich erhältlich oder können nach literaturbekannten Methoden hergestellt werden.

Weiter ist es möglich, die Carbonsäuren der allgemeinen Formel **V** in Carbonsäurechloride zu überführen und diese anschließend mit Aminen der allgemeinen Formel **IV** umzusetzen. Die Synthese von Carbonsäurechloriden erfolgt nach literaturbekannten Verfahren (siehe z. B. Houben-Weyl, Methoden der Organischen Chemie, Bd. E5/1).

(C) Reduktion der Nitrilgruppe:

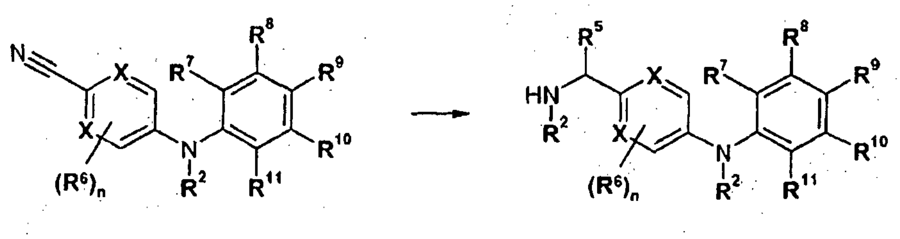

(VI) (III)

Die Reduktion eines Nitrils der allgemeinen Formel **VI** zu einem Amin der allgemeinen Formel **III,** in der der Rest $\mathbf{R^2}$ am aminischen Stickstoff Wasserstoff bedeutet und alle übrigen Reste wie vorstehend erwähnt definiert sind, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungsmittel wie Tetrahydrofuran, gegebenenfalls in Gegenwart einer Lewissäure wie Aluminiumchlorid, durchgeführt werden.

Verbindungen der allgemeinen Formel **III,** in der der Rest $\mathbf{R^2}$ am aminischen Stickstoff nicht Wasserstoff sondern beispielsweise ein Alkylrest bedeutet, lassen sich ebenfalls aus Verbindungen der allgemeinen Formel **VI** herstellen. So werden beispielsweise aus der Umsetzung eines Nitrils der allgemeinen Formel **VI** mit einem Alkylgrignard-Reagenz Ketone erhalten, die mittels reduktiver Aminierung in die Verbindungen der allgemeinen Formel **III** überführt werden

können. Die reduktive Aminierung erfolgt nach bekannten Verfahren, beispielsweise mit einem Reduktionsmittel wie Natriumtriacetoxyborhydrid, Natriumborhydrid oder Natriumcyanoborhydrid zweckmäßigerweise in einem Lösungsmittel wie Tetrahydrofuran oder Dichlormethan gegebenenfalls unter Zusatz von Essigsäure.
Alternativ können die erhaltenen Ketone auch in Oxime überführt werden. Die anschliessende Reduktion der Oxime liefert dann Verbindungen der allgemeinen Formel **III**.

(D) nucleophile aromatische Substitution oder Übergangsmetall-katalysierte Kupplung:

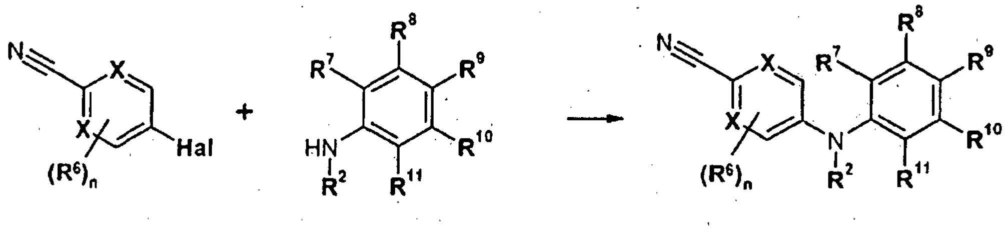

(VII) (VIII) (VI)

Die Reaktion eines Anilins der allgemeinen Formel **VIII,** in der alle Reste wie vorstehend erwähnt definiert sind, mit einem Nitril der allgemeinen Formel VII, in der **X,** $\mathbf{R_6}$ und n wie vorstehend erwähnt definiert sind, und **Hal** ein Fluor-, Chlor- oder Bromatom bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer Base wieTriethylamin, Natronlauge oder Kaliumcarbonat bei einer Temperatur von 20°C bis 160°C. Ist das Anilin der allgemeinen Formel **VIII** flüssig, kann die Reaktion auch ohne Lösungsmittel und zusätzlicher Base durchgeführt werden.
Eine alternative Methode zur Darstellung von Verbindungen der allgemeinen Formel **VI** stellt die Palladium katalysierte Reaktion eines Nitrils der allgemeinen Formel **VII,** in dem **Hal** Brom oder Chlor bedeutet, mit einem Anilin der allgemeinen Formel **VIII** dar. Reaktionsbedingungen für diese auch als Buchwald-Hartwig Reaktion bekannten Umsetzung sind aus der Literatur bekannt.

Methodenbeschreibung zur cynoBK1-Rezeptorbindung

CHO-Zellen, die den Cynomolgus B1-Rezeptor exprimieren, werden in "HAM'S F-12 Medium" kultiviert. Von konfluenten Kulturen wird das Medium entfernt, die Zellen werden mit PBS-Puffer gewaschen, abgeschabt oder mit Versene abgelöst und durch Zentrifugieren isoliert. Anschließend werden die Zellen in Suspension homogenisiert, das Homogenat zentrifugiert und resuspendiert. Nach Bestimmung des Proteingehalts werden 200 μl des Homogenats (50 bis 250 μg Protein/Assay) für 60-180 Minuten bei Raumtemperatur mit 0.5 bis 5.0 nM Kallidin (DesArg10,Leu9), [3,4-Prolyl-3,43H(N)] und ansteigenden Konzentrationen der Testsubstanz in einem Gesamtvolumen von 250 μl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCoünt NXT gemessen. Als nichtspezifische Bindung wird die gebundene Radioaktivität in Gegenwart von 1.0 μM (DesArg10) Kallidin definiert. Die Analyse der Konzentrations-Bindungskurve kann mit Hilfe einer computergestützten nichtlinearen Kurvenahpassung erfolgen, um für die Testsubstanz die entsprechenden $K_i$-Wert zu ermittelt.

Testergebnisse vom cynoBK1-Rezeptorbindungsassay:

| Beispiel Nr. | $K_i$ [nM] |
|---|---|
| (1) | 3.5 |
| (2) | 5.1 |
| (3) | 12 |
| (4) | 27 |
| (5) | 11 |

| Beispiel Nr. | $K_i$ [nM] |
|---|---|
| (6) | 3.2 |
| (7) | 30 |
| (8) | 37 |
| (9) | 6.6 |

Ein wesentliches strukturelles Merkmal der neuen Verbindungen ist die 6-Oxo-1,6-dihydro-pyridazin-4-carbonsäureamid-gruppe, die in allen erfindungsgemäßen Verbindungen enthalten ist und die im tautomeren Gleichgewicht mit der 6-Hydroxypyridazin-4-carbonsäureamid-gruppe vorliegt:

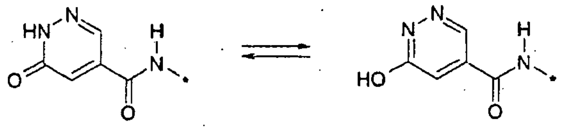

Gegenüber den Verbindungen aus dem Stand der Technik zeichnen sich die neuen Substanzen dadurch aus, dass sie eine sehr starke B1-Rezeptor blockierende Wirkung zeigen und gleichzeitig eine viel bessere metabolische Stabilität aufweisen.
Die metabolische Stabilität kann gemessen werden, indem man die Zersetzung in humanen Hepatozyten bestimmt und aus der Zersetzungsgeschwindigkeit die Clearance berechnet, die wiederum in Prozent des humanen hepatischen Blutflusses (%Qh) ausgedrückt wird. Eine Substanz mit einer hohen metabolischen Clearance (z.B. >70%Qh) wird voraussichtlich im menschlichen Organismus eine kürzere Wirkungsdauer zeigen als eine Substanz, die metabolisch stabiler ist und somit eine geringere Clearance zeigt (z.B. <30%Qh). Für eine anzustrebende lange Wirkungsdauer ist es demzufolge von großem Vorteil, wenn die Wirksubstanz eine hohe metabolische Stabilität (niedrige Clearance) aufweist. Überraschenderweise zeigen die neuen Substanzen eine niedrige Clearance in humanen Hepatozyten, wie aus folgender Tabelle hervorgeht:

| Beispiel Nr. | $K_i$ [nM] | Clearance [%Qh] |
|---|---|---|
| (1) | 3.5 | 1 |
| (3) | 12 | 7 |
| (5) | 11 | 22 |
| (6) | 3.2 | 6 |

Methodenbeschreibung zur Bestimmung der metabolischen Clearance in humanen Hepatozyten

Der metabolische Abbau der Testsubstanz wird in einer Hepatozytensuspension bestimmt. Cryokonservierte primäre humane Hepatozyten werden in einem geeigneten Inkubationsmedium (z.B. Dulbecco's modified eagle medium, DMEM), welches 5% humanes Serum enthält, inkubiert. Nach einer 30-minütigen Vorinkubation im Inkubator (37°C, 10% Kohlendioxid) werden 5µL derTestverbindung (80µM, hergestellt aus einer 2mM Stammlösung in Dimethylsulfoxid und 1:25 verdünnt mit Inkubationsmedium) zu 395µL Hepatozytensuspension gegeben (Zelldichte im Bereich 0.25-1 Million Zellen/mL, typischerweise 1 Million Zellen/mL; Endkonzentration der Testverbindung 1µM). Die Zellen werden für 6 Stunden in einem Inkubator mit Orbital-Schüttler inkubiert. Zu den Zeitpunkten 0, 0.5, 1, 2, 4 und 6h werden jeweils 25µL des Mediums entnommen. Das entnommene Medium wird mit einem Überschuss Acetonitril versetzt und 5 Minuten lang zentrifugiert. Der Überstand wird abgenommen, unter Stickstoff zur Trockne eingeengt und in einem Gemisch aus 25% Methanol und 0.1 %iger Ameisensäure aufgenommen. Die Abnahme der Konzentration der Testsubstanz im Inkubationsmedium wird mittels einer Kopplung von Flüssigkeitschromatographie an Elektrospraymassenspektrometrie bestimmt. Die lineare Phase der Abnahme der Konzentration der Testsubstanz im Medium wird zur Berechnung herangezogen. Die intrinsische Clearance wird wie folgt berechnet: CL_INTRINSIC = Dose / AUC = (C0/CD) / (AUD + clast/k) x 1000/60. C0: Anfangskonzentration in der Inkubation [µM], CD: Zelldichte der vitalen Zellen [10e6cells/mL], AUD: Fläche unter der Kurve [µM x h], clast: Konzentration des letzten Datenpunktes [µM], k: Steigung der Regressionsgeraden für die Abnahme der Testsubstanz [h-1]. Die berechnete intrinsische in vitro Clearance wird nun in die intrinsische in vivo Clearance umgerechnet:

CL_INTRINSIC_INVIVO [ml/min/kg] = (CL_INTRINSIC [µL/min/10e6cells] x Hepatozellularität [10e6 Zellen/g Leber] x Leberfaktor [g/kg Körpergerwicht]) / 1000

und es wird mittels des well-stirred Modells die abgeschätzte humane Clearance berechnet:

CL [ml/min/kg] = CL_INTRINSIC_INVIVO [ml/min/kg] x hepatischer Blutfluss [ml/min/kg] / (CL_INTRINSIC_INVIVO [ml/min/kg] + hepatischer Blutfluß [ml/min/kg]).

Folgende Parameter werden für die Berechnung verwendet: Hepatozellularität, human: 120x10e6 Zellen / g Leber; Leberfaktor, human: 25.7 g Leber/ kg Körpergewicht; Hepatischer Blutfluss, human: 21 ml/(min x kg).

**INDIKATIONEN**

Aufgrund ihrer pharmakologischen Eigenschaften eignen sich die neuen Verbindungen und deren physiologisch verträgliche Salze zur Behandlung von Krankheiten und Krankheitssymptomen, die wenigstens teilweise durch die Stimulierung von Bradykinin-B1-Rezeptoren hervorgerufen werden, oder in denen eine Antagonisierung des Bradykinin-1 Rezeptors eine Symptomverbesserung bewirken kann.

Ein weiterer Gegenstand der vorliegenden Erfindung umfasst die erfindungsgemäßen Verbindungen der eingangs erwähnten allgemeinen Formel **I** zur Verwendung als Arzneimittel.

Aufgrund ihrer pharmakologischen Wirkung eignen sich die Substanzen zur Behandlung

(a) von **akuten Schmerzen,** wie beispielsweise Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;

(b) von **Eingeweideschmerzen,** wie beispielsweise chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, Cholecystitis, Prostatitis, bei Angina pectoris, Schmerzen bei Kolon irritabile, bei nichtulzeröser Dyspepsie und bei Gästritis, Prostatitis, nichtkardialen Thoraxschmerzen und Schmerzen bei myokardialer Ischämie und Herzinfarkt;

(c) von **neuropathischen Schmerzen,** wie beispielsweise schmerzhaften Polyneuropathien, Schmerzen bei diabetischer Neuropathie, AIDS-assoziierten neuropathischen Schmerzen, bei nicht-herpes-assoziierter Neuralgie, bei Postzoster Neuralgie, bei Nervenverletzungen, bei Schädel-Hirn-Trauma, Schmerzen bei Nervenschädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen, sowie zentralem Schmerz wie z.B. nach Schlaganfall, Rückenmarksverletzungen oder Tumoren;

(d) von **entzündlichen / Schmerzrezeptor-vermittelten Schmerzen** im Zusammenhang mit Erkrankungen wie beispielsweise Osteoarthritis, rheumatoider Arthritis, rheumatischem Fieber, Tendo-Synovitis, Bursitis, Sehnenentzündungen, Gicht und Gicht-Arthritis, traumatische Arthritiden, Vulvodynie, Schädigungen und Erkrankungen der Muskeln und Faszien, juveniler Arthritis, Spondylitis, Psoriasis-Arthritis, Myositiden, Zahnerkrankungen, Influenza und anderen Virusinfektionen wie Erkältungen, systemischer Lupus erythematodes oder Schmerzen bei Verbrennungen;

(e) von **Tumorschmerzen** im Zusammenhang mit Krebserkrankungen, wie beispielsweise lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;

(f) von **Kopfschmerz-Erkrankungen** unterschiedlicher Ursache, wie beispielsweise Cluster-Kopfschmerz, Migräne (mit oder ohne Aura) und Spannungskopfschmerz;

(g) von Schmerzzuständen gemischter Ursache, wie beispielsweise chronische Rückenschmerzen, einschließlich Lumbago, oder Fibromyalgie.

Weiterhin eigenen sich die Verbindungen zur Behandlung

(h) von entzündlichen Erkrankungen oder Entzündungserscheinungen bei Sonnenbrand und Verbrennungen, von Zahnfleischentzündungen, Ödemen nach Traumata durch Verbrennungen, Hirnödemen und Angioödemen, Darmerkrankungen einschließlich Morbus Crohn und Colitis ulzerosa, Reizdarmsyndröm, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Zerrungen und Frakturen, sowie muskuloskeletale Erkrankungen mit Entzündungserscheinungen wie akutes rheumatisches Fieber, Polymyalgia Rheumatica, reaktive Arthritiden, Rheumatiode Arthritis, Spondylarthritiden, aber auch Oseoarthritis, sowie entzündliche Bindegewebserkrankungen anderer Genese, und Kollagenosen jeglicher Genese wie systemischer Lupus Erythematodes, Sklerodermie, Polymyositis, dermatomyositis, Sjögren Syndrom, Morbus Still oder Felty Syndrom;
(i) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
(j) von chronischer Bronchitis sowie chronisch obstruktiver Lungen-Erkrankung (COPD) einschließlich Lungenemphysem, virale oder bakterielle Exazerbation von chronischer Bronchitis oder chronisch obstruktiver Bronchitis, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose, Byssinose, exogen allergische Alveolitiden, Lungenfibrose, Bronchiektasien, Lungenerkrankungen bei alpha1-Antritrypsinmangel und Husten;
(k) von Diabetes Mellitus und dessen Folgen (wie beispielsweise diabetische Vaskulopathie, diabetische Neuropathie, diabetische Retinopathie) und von diabetischen Symptomen bei Insulitis (beispielsweise Hyperglycämie, Diurese, Proteinurie und erhöhte renale Nitrit- und Kallikrein-Exkretion);
(l) von Sepsis und septischem Schock nach bakteriellen Infektionen oder nach Trauma;
(m) von Juckreiz (Pruritus) verursachenden Syndromen und allergischen Hautreaktionen;
(n) von Verletzungen des Zentralnervensystems;
(o) von Wunden und Gewebeschädigungen;
(p) von benigner Prostata-Hyperplasie und hyperaktiver Blase;
(q) von vaskulären Erkrankungen wie Panarteriitis nodosa, Polyarthritis nodosa, Periarteriitis nodosa, Arteriitis temporalis, Wegnersche Granulomatose, Riesenzellartriitis, Arteriosklerose, Erythema nodosum;
(r) von Störungen der Motilität oder Spasmen von respiratorischen, genito-urinalen, gastro-intestinalen inclusive biliärer, oder vaskulären Strukturen und Organen;
(s) von post-operativem Fieber;
(t) zur Behandlung und Vorbeugung von cardiovasculären Erkrankungen, wie beispielsweise Bluthochdruck und verwandte Erkrankungen;
(u) zur Behandlung und Vorbeugung von Krebs und verwandten Erkrankungen;
(v) zur Behandlung und Vorbeugung von psychatrischen Erkrankungen, wie beispielsweise Depressionen;
(w) zur Behandlung und Vorbeugung von Harninkontinenz und verwandten Erkrankungen;
(x) zur Behandlung und Vorbeugung von krankhaftem Übergewicht und verwandten Erkrankungen;
(y) zur Behandlung und Vorbeugung von Artheriosklerose und verwandten Erkrankungen; und
(z) zur Behandlung und Vorbeugung von Epilepsie.

Die Substanzen eignen sich zur kausalen Behandlung im Sinne einer Verlangsamung oder Unterbrechung des Voranschreitens chronisch progredienter Erkrankungen, insbesondere von Osteoarthritis, Rheumatoider Arthritis und Spondylarthritiden.

Ein weiterer Gegenstand der vorliegenden Erfindung umfasst die Verwendung der erfindungsgemäßen Verbindungen der eingangs erwähnten allgemeinen Formel **I** zur Herstellung eines Arzneimittels für eine therapeutische Anwendung in den voranstehend genannten Indikationen.

Bevorzugt werden die erfindungsgemäßen Verbindungen der allgemeinen Formel **I** zur Behandlung von Osteoarthritis, Rheumatioder Arthritis oder COPD eingesetzt.

Unter dem Ausdruck "Behandlung" oder "Therapie" ist eine therapeutische Behandlung von Patienten mit manifester, akuter oder chronischer Indikation zu verstehen, wobei einerseits die symptomatische (palliative) Behandlung zur Linderung der Krankheitssymptome und andererseits die kausale oder kurative Behandlung der Indikation mit dem Ziel, den pathologischen Zustand zu beenden, den Schweregrad des pathologischen Zustands zu vermindern oder die Progression des pathologischen Zustands zu verzögern, abhängig von der Art oder Schwere der Indikation, eingeschlossen sind.

Ein weiterer Gegenstand der vorliegenden Erfindung ist die Verwendung einer Verbindung der allgemeinen Formel **I** zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen, Kopfschmerz-Erkrankungen und Schmerzzuständen gemischter Ursache sowie weiterer der voranstehend genannten Erkrankungen. Dabei ist die Verwendung dadurch gekennzeichnet, dass sie die Verabreichung einer wirksamen Menge einer Verbindung der allgemeinen Formel **I** oder eines physiologisch verträglichen Salzes davon an einen Patienten beinhaltet, der einer solchen Behandlung bedarf.

Unter dem Begriff "Patient" wird vorzugsweise ein Mensch verstanden.

Neben derEignung als Humantherapeutika, sind diese Substanzen auch nützlich in derveterinärmedizinischen Therapie von Haustieren, exotischen Tieren und Nutztieren.

## KOMBINATIONEN

Zur Behandlung von Schmerzen kann es vorteilhaft sein, die erfindungsgemäßen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemäßen Verbindungen kombiniert werden.

Für eine Kombinationstherapie kommen beispielsweise die folgenden Verbindungen in Frage:

Nicht-steroidale Antirheumatika (NSAR), wie beispielsweise Propionsäure-Derivate, die ausgewählt sein können aus der Gruppe bestehend aus Alminoprofen, Bucloxinsäure, Carprofen, Fenoprofen, Ibuprofen, Ketoprofen, Naproxen, Oxaprozin, Pirprofen, Pranoprofen und Tiaprofensäure; Essigsäure-Derivate, die ausgewählt sein können aus der Gruppe bestehend aus Indomethacin, Acemetacin, Alcofenac, Isoxepac, Sulindac und Tolmetin; Fenaminsäure-Derivate, die ausgewählt sein können aus der Gruppe bestehend aus Meclofenaminsäure, Mefenaminsäure und Tolfenaminsäure; Biphenyl-Carboxylsäure-Derivate; Oxicame, die ausgewählt sein können aus der Gruppe bestehend aus Meloxicam, Piroxicam und Tenoxicam; Salicylsäure-Derivate, die ausgewählt sein können aus der Gruppe bestehend aus Acetylsalicylsäure und Sulfasalazin; Pyrazolone, die ausgewählt sein können aus der Gruppe bestehend ausApazon und Feprazon); Coxibe, die ausgewählt sein können aus der Gruppe bestehend aus Celecoxib und Etoricoxib.

Opiat Rezeptor Agonisten, die beispielsweise ausgewählt sein können aus der Gruppe bestehend aus wie Morphin, Darvon, Tramadol und Buprenorphin.

Cannabinoid Agonisten, wie beispielsweise GW-1000.

Natriumkanalblocker, die beispielsweise ausgewählt sein können aus der Gruppe bestehend aus Carbamazepin, Mexiletin, Pregabalin, Tectin und Ralfinamide.

N-Typ Calciumkanalblocker, wie beispielsweise Ziconotid.

Serotonerge und noradrenerge Modulatoren, die beispielsweise ausgewählt sein können aus der Gruppe bestehend aus Duloxetin und Amitriptylin.

Corticosteroide, die beispielsweise ausgewählt sein können aus der Gruppe bestehend aus Betamethasön, Budesonid, Cortison, Dexamethason, Hydrocortison, Methylprednisolon, Prednisolon, Prednison und Triamcinolon.

Histamin H1-Rezeptorantagonisten, die beispielsweise ausgewählt sein können aus der Gruppe bestehend aus Brompheniramin, Chlorpheniramin, Dexchlorpheniramin, Triprolidin, Clemastin, Diphenhydramin, Diphenylpyralin, Tripelennamin, Hydroxyzin, Promethazin, Trimeprazin, Azatadin, Cyproheptadin, Antazolin, Pheniramin, Pyrilamin, Loratadin, Cetirizin, Desloratadin, Fexofenadin und Levocetirizin.

Leukotrien Antagonisten und 5-Lipoxygenasehemmer, die beispielsweise ausgewählt sein können aus der Gruppe bestehend aus Zafirlukast, Montelukast, Pranlukast und Zileuton.

Lokalanästhetika, die beispielsweise ausgewählt sein können aus der Gruppe bestehend aus Ambroxol und Lidocain.

TRPV1 Antagonisten, die beispielsweise ausgewählt sein können aus der Gruppe bestehend aus AZD-1386, JTS-653 und PHE-377.

Nikotinrezeptor Agonisten, wie beispielsweise A-366833.

P2X3-Rezeptor Antagonisten, wie beispielsweise A-317491.

anti-NGF Antikörper und NGF Antagonisten, die beispielsweise ausgewählt sein können aus der Gruppe bestehend aus JNJ-42160443 und PPH 207.

NK1 und NK2 Antagonisten, wie beispielsweise CP-728663.

NMDA Antagonisten, die beispielsweise ausgewählt sein können aus der Gruppe bestehend aus CNS-5161, AZ-756 und V-3381.

Kaliumkanal Modulatoren, wie beispielsweise CL-888.

GABA Modulatoren, wie beispielsweise Baclofen.

Anti-Migräne Therapeutika, die beispielsweise ausgewählt sein können aus der Gruppe bestehend aus Sumatriptan, Zolmitriptan, Naratriptan und Eletriptan.

Zur Behandlung von einer oder mehrerer der voranstehend genannten Atemwegserkrankungen kann es vorteilhaft sein, die erfindungsgemäßen Verbindungen der allgemeinen Formel **I** mit anderen Wirkstoffen zur Behandlung von Atemwegserkrankungen zu kombinieren. Stehen zur Behandlung der Ursache der Atemwegserkrankungen geeignete Wirkstoffe zur Verfügung, so können diese mit den erfindungsgemäßen Verbindungen kombiniert werden.

Gegebenenfalls können die Verbindungen der allgemeinen Formel **I** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Rezeptor (CysLT1, CysLT2, CysLT3) Antagonisten, Inhibitoren von MAP Kinasen wie zum Beispiel p38, ERK1, ERK2, JNK1, JNK2, JNK3 oder SAP, LTB4-Rezeptor (BLT1, BLT2) Antagonisten, EGFR-Hemmern, H1-Rezeptor Antagonisten, Antihistaminika, H4-Rezeptor Antagonisten, PAF-Antagonisten und PI3-Kinase Inhibitoren CXCR1 und/ oder CXCR2, Rezeptor Antagonisten und Substanzen gegen Husten.

Die Verbindungen der allgemeinen Formel **I** können auch in Form von Zwei- oder Dreifachkombinationen davon eingesetzt werden, wie beispielsweise Kombinationen von Verbindungen der Formel **I** mit ein oder zwei Verbindungen aus der Gruppe bestehend aus

- Betamimetika, Corticosteroiden, PDE4-Inhibitoren, EGFR-Hemmer und LTD4-Antagonisten,
- Anticholinergika, Betamimetika, Corticosteroiden, PDE4-Inhibitoren, EGFR-Hemmern und LTD4-Antagonisten,
- PDE4-Inhibitoren, Corticosteroiden, EGFR-Hemmern und LTD4-Antagonisten,
- EGFR-Hemmern, PDE4-inhibitoren und LTD4-Antagonisten,
- EGFR-Hemmern und LTD4-Antagonisten,
- CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate, die in der WO 2006/120176 genannt sind, und SYK-Inhibitoren (spleen tyrosine kinase-Inhibitoren),
- Anticholinergika, Betamimetika, Corticosteroiden, PDE4-Inhibitoren und MRP4-Inhibitoren.

Auch die Kombinationen dreier Wirkstoffe je einer der oben genannten Verbindungsklassen ist Bestandteil der Erfindung.

Als **Betamimetika** gelangen erfindungsgemäß vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol und Zinterol oder

- 6-Hydroxy-8-{1-hydroxy-2-[2-(4-methoxy-phenyl)-1,1-dimethyl-ethylamino]-ethyl}-4H-benzo[1,4]oxazin-3-on,
- 8-{2-[2-(2,4-Difluor-phenyl)-1,1-dimethyl-ethylaminoj-1-hydroxy-ethyl}-6-hydroxy-4H-benzo[1,4]oxazin-3-on,
- 8-{2-[2-(3,5-Difluor-phenyl)-1,1-dimethyl-ethylamino]-1-hydroxy-ethyl}-6-hydroxy-4H-benzo[1,4]oxazin-3-on,
- 8-{2-[2-(4-Ethoxy-phenyl)-1,1-dimethyl-ethylamino]-1-hydroxy-ethyl}-6-hydroxy-4H-benzo[1,4]oxazin-3-on,
- 8-{2-[2-(4-Fluor-phenyl)-1,1-dimethyl-ethylamino]-1-hydroxy-ethyl}-6-hydroxy-4H-benzo[1,4]oxazin-3-on,
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[3-(4,4-Diethyl-6-methoxy-2-oxo-4H-benzo[d][1,3]oxazin-1-yl)-1,1-dimethyl-propylamino]-1-hydroxyethyl}=2-hydroxy-phenyl)-methansulfonamid,
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid,
- 8-{2-[1,1-Dimethyl-3-(2-oxo-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on,
- N-[2-Hydroxy-5-((1R)-1-hydroxy-2-{2-[4-(2-hydroxy-2-phenyl-ethylamino)-phenyl]-ethylamino}-ethyl)-phenyl]-formamid
- 8-Hydroxy-5-((1R)-1-hydroxy-2-{2-[4-(6-methoxy-biphenyl-3-ylamino)-phenyl]-ethylamino}-ethyl)-1H-chinolin-2-on,

- 8-Hydroxy-5-[(1R)-1-hydroxy-2-(6-phenethylamino-hexylamino)-ethyl]-1H-chinolin-2-on,
- 5-[(1R)-2-(2-{4-[4-(2-Amino-2-methyl-propoxy)-phenylamino]-phenyl}-ethylamino)-1-hydroxy-ethyl]-8-hydroxy-1H-chinolin-2-on,
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff,
- 4-((1R)-2-{6-[2-(2,6-Dichlor-benzyloxy)-ethoxy]-hexylamino}-1-hydroxy-ethyl)-2-hydroxymethyl-phenol,
- 3-(4-{6-[(2R)-2-Hydroxy-2-(4-hydroxy-3-hydroxymethyl-phenyl)-ethylamino)-hexyloxy}-butyl)-benzenesulfonamid,
- 3-(3-{7-[(2R)-2-Hydroxy-2-(4-hydroxy-3-hydroxymethyl-phenyl)-ethylamino]-heptyloxy}-propyl)-benzenesulfonamid,
- 4-((1R)-2-{6-[4-(3-Cyclopentanesulfonyl-phenyl)-butoxy]-hexylamino}-1-hydroxy-ethyl)-2-hydroxymethyl-phenol,
- *N*-1-Adamantanyl-2-{3-[(2R)-2-({(2R)-2-hydroxy-2-[4-hydroxy-3-(hydroxymethyl)-phenyl]ethyl}amino)propyl]phenyl}acetamid,
- (1R)-5-{2-[6-(2,2-Difluor-2-phenyl-ethoxy)-hexylamino]-1-hydroxy-ethyl}-8-hydroxy-1H-chinolin-2-on
- (R,S)-4-(2-{[6-(2,2-Difluor-4-phenylbutoxy)hexyl]amino}-1-hydroxy-ethyl)-2-(hydroxyl-methyl)phenol,
- (R,S)-4-(2-{[6-(2,2-Difluor-2-phenylethoxy)hexyl]amino}-1-hydroxy-ethyl)-2-(hydroxyl-methyl)phenol,
- (R,S)-4-(2-{[4,4-Difluor-6-(4-phenylbutoxy)hexyl]amino}-1-hydroxy-ethyl)-2-(hydroxyl-methyl)phenol,
- (R,S)-4-(2-{[6-(4,4-Difluor-4-phenylbutoxy)hexyl]amino}-1-hydroxy-ethyl)-2-(hydroxyl-methyl)phenol,
- (R,S)-5-(2-{[6-(2,2-Difluor-2-phenylethoxy)hexyl]amino}-1-hydroxy-ethyl)-8-hydroxy-chinolin-2(1H)-on,
- (R,S)-[2-({6-[2,2-Difluor-2-(3-methylphenyl)ethöxy]hexyl}amino)-1-hydroxyethyl]-2-(hydroxymethyl)phenol,
- 4-(1R)-2-{[6-(2,2-Difluor-2-phenylethoxy)hexyl]amino}-1-hydroxyethyl)-2-(hydroxyl-methyl)phenol,
- (R,S)-2-(Hydroxymethyl)-4-(1-hydroxy-2-{[4,4,5,5-tetrafluor-6-(3-phenylpropoxy)-hexyl]amino}ethyl)phenol,
- (R,S)-[5-(2-{[6-(2,2-Difluor-2-phenylethoxy)hexyl]amino}-1-hydroxy-ethyl)-2-hydroxy-phenyl]formamid,
- (R,S)-4-[2-({6-[2-(3-Bromophenyl)-2,2-difluoroethoxy]hexyl}amino)-1-hydroxyethyl]-2-(hydroxymethyl)phenol,
- (R, S)-N-[3-(1,1-Difluor-2-{[6-({2-hydroxy-2-[4-hydroxy-3-(hydroxymethyl)phenyl]-ethyl}amino)hexyl]oxy}ethyl)phenyl]-harnstoff,
- 3-[3-(1,1-Difluor-2-{[6-({2-hydroxy-2-[4-hydroxy-3-(hydroxymethyl) phenyl]ethyl}-amino)hexyl]oxy}ethyl)phenyl]imidazolidin-2,4-dion,
- (R,S)-4-[2-({6-[2,2-Difluor-2-(3-methoxyphenyl)ethoxy]hexyl}amino)-1-hydroxyethyl]-2-(hydroxymethyl)phenol,
- 5-((1R)-2-{[6-(2,2-Difluor-2-phenylethoxy)hexyl]amino}-1-hydroxyethyl)-8-hydroxychinolin-2(1H)-on,
- 4-((1R)-2-{[4,4-Difluor-6-(4-phenylbutoxy)hexyl]amino}-1 hydroxy-ethyl)-2-(hydroxyl-methyl)phenol,
- (R,S)-4-(2-{[6-(3,3-Difluor-3-phenylpropoxy)hexyl]amino}-1-hydroxy-ethyl)-2-(hydroxyl-methyl)phenol,
- (R,S)-(2-{[6-(2,2-Difluor-2-phenylethoxy)-4,4-difluorohexyl]amino}-1-hydroxyethyl)-2-(hydroxymethyl)phenol,
- (R,S)-4-(2-([6-(2,2-Difluor-3-phenylpropoxy)hexyl]amino)-1-hydroxyethyl)-2-(hydroxyl-methyl)phenol,
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}-propionamid,
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid,
- 7-[2-(2-{3-[2-(2-Chlor-phenyl)-ethylamino]-propylsulfanyl}-ethylamino)-1-hydroxyethyl]-4-hydroxy-3H-benzothiazol-2-on,

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Anticholinergika** gelangen erfindungsgemäß vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X- können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.
Weitere Anticholinergika können ausgewählt sein aus der Gruppe bestehend aus

- 2,2-Diphenylpropionsäuretropenolester-Methobromid,
- 2,2-Diphenylpropionsäurescopinester-Methobromid,
- 2-Fluor-2,2-Diphenylessigsäurescöpinester-Methobromid,
- 2-Fluor-2,2-Diphenylessigsäuretropenolester-Methobromid,
- 3,3',4,4'-Tetrafluorbenzilsäuretropenolester-Methobromid,
- 3,3',4,4'-Tetrafluorbenzilsäurescopinester-Methobromid,
- 4,4'-Difluorbenzilsäuretropenolester-Methobromid,
- 4,4'-Difluorbenzilsäurescopinester-Methobromid,
- 3,3'-Difluorbenzilsäuretropenolester-Methobromid,
- 3,3'-Difluorbenzilsäurescopiriester-Methobromid,
- 9-Hydroxy-fluoren-9-carbonsäuretropenolester-Methobromid,
- 9-Fluor-fluoren-9-carborisäuretropenolester-Methobromid,
- 9-Hydroxy-fiuoren-9-carbonsäurescopinester-Methobromid,
- 9-Fluor-fluoren-9-carbonsäurescopinester-Methobromid,
- 9-Methyl-fluoren-9-carbonsäuretropenolester-Methobromid,
- 9-Methyl-fluoren-9-carbonsäurescopinester-Methobromid,
- Benzilsäurecyclopropyltropinester-Methobromid,
- 2,2-Diphenylpropionsäurecyclopropyltropinester-Methobromid,
- 9-Hydroxy-xanthen-9-carbonsäurecyclopropyltropinester-Methobromid,
- 9-Methyl-fluoren-9-carbonsäurecyclopropyltropinester-Methobromid,
- 9-Methyl-xanthen-9-carbonsäurecyclopröpyltropinester-Methobromid,
- 9-Hydroxy-fluoren-9-carbonsäurecyclopropyltropinester-Methobromid,
- 4,4'-Difluorbenzilsäuremethylestercyclopropyltropinester-Methobromid,
- 9-Hydroxy-xanthen-9-carbonsäuretropenoiester-Methobromid,
- 9-Hydroxy-xanthen-9-carbönsäurescopinester-Methobromid,
- 9-Methyl-xanthen-9-carbonsäuretropenolester-Methobromid,
- 9-Methyl-xanthen-9-carbonsäurescopinester-Methobromid,
- 9-Ethyl-xanthen-9-carbonsäuretropenolester-Methobromid,
- 9-Difluormethyl-xanthen-9-carbonsäuretropenolester-Methobromid, und
- 9-Hydroxymethyl-xanthen-9-carbonsäurescopinester-Methobromid.

Die vorstehend genannten Verbindungen sind im Rahmen der vorliegenden Erfindung auch als Salze,einsetzbar, in denen statt des Methobromids, die Salze Metho-X zur Anwendung gelangen, wobei X die vorstehend für $X^-$ genannten Bedeutungen haben kann.

- Als **Corticosteroide** gelangen erfindungsgemäß vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon und Tipredane oder Pregna-1,4-dien-3,20-dion, 6-Fluor-11-hydroxy-16,17-[(1-methylethyliden)-bis(oxy)]-21-[[4-[(nitrooxy)methyl]benzoyl]oxy]-, (6-alpha,11-beta,16-alpha)-(9Cl) (NCX-1024)
- 16,17-Butylidendioxy-6,9-difluor-11-hydroxy-17-(methylthio)androst-4-en-3-on (RPR-106541),
- 6,9-Difluor-17-[(2-furanylcarbonyl)oxy]-11-hydroxy-16-methyl-3-oxo-androsta-1,4-dien-17-carbothionsäure-(S)-fluormethylester,
- 6,9-Difluor-11-hydroxy-16-methyl-3-oxo-17-propionyloxy-androsta-1,4-dien-17-carbo-thionsäure (S)-(2-oxo-tetrahydrofuran-3S-yl)ester, und
- 6-alpha,9-alpha-Difluor-11-beta-hydroxy-16alpha-methyl-3-oxo-17alpha-(2,2,3,3-tetramethylcyclopropylcarbonyl)oxy-androsta-1,4-dien-17beta-carbonsäure-cyanomethylester,

gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als **PDE4-Inhibitoren** gelangen erfindungsgemäß vorzugsweise Verbindungen zur Anwendung, die ausgewählt sind aus der Gruppe bestehend aus Enprofyllin, Theophyllin, Roflumilast, Ariflo (Cilomilast), TofimilastPumafentrin, Lirimilast, Apremilast, Arofyllin, Atizoram, Oglemilast und Tetomilast oder

- 5-[(N-(2,5-Dichlor-3-pyridinyl)-carboxamid]-8-methoxy-chinolin (D-4418),

- 5-N-(3,5-Dichlor-1-oxido-4-pyridinyl)-carboxamid]-8-methoxy-2-(trifluormethyl)-chinolin (D-4396 (Sch-351591)),N-(3,5-Dichlorpyrid-4-yl)-[1-(4-fluorbenzyl)-5-hydroxy-indol-3-yl]glyoxylsäureamid (AWD-12-281 (GW-842470)), 9-[(2-Fluorphenyl)methyl]-N-methyl-2-(trifluormethyl)-9H-purin-6-amin (NCS-613),
- 4-[(2R)-2-[3-(Cyclopentyloxy)-4-methoxyphenyl]-2-phenylethyl]-pyridin (CDP-840),
- N-[(3R)-3,4,6,7-Tetrahydro-9-methyl-4-oxo-1-phenylpyrrolo[3,2,1-jk][1,4]benzodiazepin-3-yl]-4-pyridincarboxamid (PD-1168787),
- 4-[6,7-Diethoxy-2,3-bis(hydroxymethyl)-1-naphthalenyl]-1-(2-methoxyethyl)-2(1H)-pyridinon (T-440),
- 2-[4-[6,7-Diethoxy-2,3-bis(hydroxymethyl)-1-naphthalenyl]-2-pyridinyl]-4-(3-pyridinyl)1(2H)-phthalazinon (T-2585),
- (3-(3-Cyclopenyloxy-4-methoxybenzyl)-6-ethylamino-8-isopropyl-3H-purin (V-11294A),
- beta-[3-(Cyclopentyloxy)-4-methoxyphenyl]-1,3-dihydro-1,3-dioxo-2H-isoindol-2-propanamid (CDC-801),
- Imidazo[1,5-a]pyrido[3,2-e]pyrazin-6(5H)-on, 9-ethyl-2-methoxy-7-methyl-5-propyl-(D-22888),
- 5-[3-(cyclopentyloxy)-4-methoxyphenyl]-3-[(3-methylphenyl)methyl]-, (3S,5S)-2-Piperidinon (HT-0712),
- 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141),
- N-(3,5-Dichlor-1-oxo-pyridin-4-yl)-4-difluormethoxy-3-cyclopropylmethoxybenzamid,
- (-)p-[(4*a*R*,10*b*S*)-9-Ethoxy-1,2,3,4,4a,10b-hexahydro-8-methoxy-2-methylbenzo[s]-[1,6]naphthyridin-6-yl]-N,N-diisopropylbenzamid,
- (R)-(+)-1-(4-Brombenzyl)-4-[(3-cyclopentyloxy)-4-methoxyphenyl]-2-pyrrolidon,
- 3-(Cyclopentyloxy-4-methoxyphenyl)-1-(4-N'-[N-2-cyano-S-methyl-isothioureido]-benzyl)-2-pyrrolidon,
- cis[4-Cyano-4-(3-cyclopentyloxy-4-methoxyphenyl)cyclohexan-1-carbonsäure],
- 2-Carbomethoxy-4-cyano-4-(3-cyclopropylmethoxy-4-difluoromethoxyphenyl)-cyclohexan-1-on,
- cis[4-Cyano-4-(3-cyclopropylmethoxy-4-difluormethoxyphenyl)cyclohexan-1-ol],
- (R)-(+)-Ethyl[4-(3-cyclopentyloxy-4-methoxyphenyl)pyrrolidin-2-yliden]acetate,
- (S)-(-)-Ethyl[4-(3-cyclopentyloxy-4-methoxyphenyl)pyrrolidin-2-yliden]acetate,
- 9-Cyclopentyl-5,6-dihydro-7-ethyl-3-(2-thienyl)-9H-pyrazolo[3,4-c]-1,2,4-triazolo-[4,3-a]pyridin und
- 9-Cyclopentyl-5,6-dihydro-7-ethyl-3-(*tert*-butyl)-9*H*-pyrazolo[3,4-c]-1,2,4-triazolo-[4,3-a]pyridin,

gegebenenfalls in Form ihrer Racemate, Enantiomere, Diastereomere und gegebenenfalls in Form ihrerpharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **EGFR-Hemmer** gelangen erfindungsgemäß vorzugsweise Verbindungen zur Anwendung, die ausgewählt sind aus der Gruppe bestehend ausCetuximab, Trastuzumab, Panitumumab (= ABX-EGF), Mab ICR-62, Gefitinib, Canertinib und Erlotinib oder

- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-((3=Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-büten-1-yl]amino}-7-cyclopropylmethoxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-[(S)-tetrahydrofuran-3-yl)oxy]-chinazolin,
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin,
- 4-[(3-Chlor-4-fluor-phenyl)amino]-6-[2-((S)-6-methyl-2-oxo-morpholin-4-yl)-ethoxy]-7-methoxy-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-y)]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,

- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-büten-1-yl}amino)-7-cyclopropylmethoxy-chinazolin,
- 4-((3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3-Chlor-4-fluorphenyl)amino]-6-{[4-(N,N-dimethylamiho)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)-amino]-chinazolin,
- 4-[(R)-(1-Phenyl-ethyl)amino]-6-(4-hydroxy-phenyl)-7H-pyrrolo[2,3-d]pyrimidin,
- 3-Cyano-4-[(3-chlor-4-flüorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methänsulfonyl-ethyl)amino]-methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5;5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(*tert*-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-flubr-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonyl-amino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,

- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin,
- 4-[(3-Chlor-4-fluor-phenyl)arnino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(*tert*-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phehyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)-carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-metbyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin ;

- [4-[(3-Chlor-4-fluorphenyl)amino]-6-{[4-(homomorpholin-4-yl)-1-oxo-2-buten-1-yl]-amino}-7-[(S)-(tetrahydrofuran-3-yl)oxy]-chinazolin,
- 4-[(3-Chlor-4-fluorphenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluorphenyl)amino]-7-[2-((S)-6-methyl-2-oxo-morpholin-4-yl)-ethoxy]-6-[(vinylcarbonyl)amino]-chinazolin,
- 4-[(3-Chlor-4-fluorphenyl)amino]-7-[4-((R)-6-methyl-2-oxo-morpholin-4-yl)-butyloxy]-6-[(vinylcarbonyl)amino]-chinazolin,
- 4-[(3-Chlor-4-fluorphenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxy-carbonyl)methyl]amino}-1-oxo-2-buten-1-yl)amino]-7-cyclopropylmethoxy-chinazolin,

gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

- Als **LTD4-Rezeptor Antagonisten** gelangen erfindungsgemäß vorzugsweise Verbindungen zur Anwendung, die ausgewählt sind aus der Gruppe bestehend aus Montelukast, Pranlukast und Zafirlukast, oder(E)-8-[2-[4-[4-(4-Fluorphenyl)butoxy]phenyl]ethenyl]-2-(1H-tetrazol-5-yl)-4H-1-benzopyran-4-on (MEN-91507),
- 4-[6-Acetyl-3-[3-(4-acetyl-3-hydroxy-2-propylphenylthib)propoxy]-2-propylphenoxy]-buttersäure (MN-001),
- 1-(((R)-(3-(2-(6,7-Difluor-2-chinolinyl)ethenyl)phenyl)-3-(2-(2-hydroxy-2-propyl)phenyl)-thio)methylcyclopropanessigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure und
- [2-[[2-(4-*tert*-Butyl-2-thiazolyl)-5-benzofuranyl]oxymethyl]phenyl]essigsäure,

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als **Histamin H1 Rezeptor Antagonisten** gelangen erfindungsgemäß vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Histamin H4 Rezeptor Antagonisten** gelangen erfindungsgemäß vorzugsweise Verbindungen zur Anwendung wie beispielsweise (5-Chlor-1H-indol-2-yl)-(4-methyl-1-piperazinyl)-Methanon (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als **MAP Kinase Inhibitoren** gelangen erfindungsgemäß vorzugsweise Verbindungen zur Anwendung, die ausgewählt sind aus der Gruppe bestehend aus:

- Bentamapimod (AS-602801)

- Doramapimod,
- 5-Carbamoylindole (SD-169),
- 6-[(Aminocarbonyl)(2,6-difluorphenyl)amino]-2-(2,4-difluorphenyl)-3-pyridincarboxamid (VX-702),
- alpha-[2-[[2-(3-Pyridinyl)ethyl]amino]-4-pyrimidinyl]-2-benzothiazoleacetonitril (AS-601245),
- 9,12-Epoxy-1H-diindolo[1,2,3-fg:3',2',1'-kl]pyrrolo[3,4-i][1,6]benzodiazocin-10-Carboxylsäure (CEP-1347), und
- 4-[3-(4-Chlorphenyl)-5-(1-methyl-4-piperidinyl)-1H-pyrazol-4-yl]-pyrimidin (SC-409),

gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **Neurokinin (NK1 oder NK2) Antagonisten** gelangen erfindungsgemäß vorzugsweise Verbindungen zur Anwendung, die ausgewählt sind aus der Gruppe bestehend aus: Saredutant, Nepadutant und Figopitant, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **Substanzen gegen Husten** gelangen erfindungsgemäß vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter **CXCR1 oder CXCR2 Antagonisten** gelangen erfindungsgemäß vorzugsweise Verbindungen zur Anwendung, wie z.B. 3-[[3-[(Dimethylamino)-carbonyl]-2-hydroxyphenyl]amino]-4-[[(R)-1-(5-methylfuran-2-yl)propyl]amino]cyclobut-3-en-1,2-dion (SCH-527123),
gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Die zur Erzielung einer schmerzstillenden, Wirkung erforderliche Dosierung beträgt bei intravenöser Gäbe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils einmal bis dreimal täglich. Die erfindungsgemäß hergestellten Verbindungen können, intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristallinerZellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylsfearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

## EXPERIMENTELLER TEIL

Für die hergestellten Verbindungen liegen in der Regel Massenspektren und/oder $^1$H-NMR-Spektren vor. Die bei den Fliessmittein angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX™, 35 bis 70 μm) oder Alox (E. Merck, Darmstadt, Aluminiumoxid 90 standardisiert, 63 bis 200 μm, Artikel-Nr: 1.01097.9050) verwendet.

In den Versuchsbeschreibungen werden die folgenden Abkürzungen verwendet:

| | |
|---|---|
| DC | Dünnschichtchromatogramm |
| DMSO | Dimethylsulfoxid |
| RP | Reverse Phase |
| $R_t$ | Retentionszeit |
| *tert* | tertiär |
| TBTU | 2-(1*H*-Benzotriazol-1-yl)-1,1,3,3-tetramethyluronium-tetrafluorborat |
| THF | Tetrahydrofuran |

Folgende analytische HPLC-Methoden wurden verwendet:

| | | |
|---|---|---|
| Methode 1: | Säule: | Interchim Strategy C18, 5 μM, 4.6 x 50 mm |
| | Detektion: | 220 - 320 nm |

(fortgesetzt)

| | | | | |
|---|---|---|---|---|
| | Fließmittel A: | Wasser / 0.1% Essigsäure | | |
| | Fließmittel B: | Acetonitril | | |
| | Gradient: | | | |
| | Zeit in min | %A | %B | Flussrate in mL/min |
| | 0.0 | 95.0 | 5.0 | 3.0 |
| | 0.3 | 95.0 | 5.0 | 3.0 |
| | 2.0 | 2.0 | 98.0 | 3.0 |
| | 2.4 | 2.0 | 98.0 | 3.0 |
| | 2.45 | 95.0 | 5.0 | 3.0 |
| | 2.8 | 95.0 | 5.0 | 3.0 |
| Methode 2: | Säule: | Merck Cromolith Flash RP18e, 4.6 x 25 mm | | |
| | Fließmittel A: | Wasser / 0.1 % Ameisensäure | | |
| | Fließmittel B: | Acetonitril / 0.1% Ameisensäure | | |
| | Gradient: | | | |
| | Zeit in min | %A | %B | Flussrate in mL/min |
| | 0.0 | 90.0 | 10.0 | 1.6 |
| | 2.7 | 10.0 | 90.0 | 1.6 |
| | 3.0 | 10.0 | 90.0 | 1.6 |
| | 3.3 | 90.0 | 10.0 | 1.6 |
| Methode 3: | Säule: | YMC-Pack ODS-AQ, 3 μM, 4.6 x 75 mm | | |
| | Fließmittel A: | Wasser / 0.15% Ameisensäure | | |
| | Fließmittel B: | Acetonitril | | |
| | Gradient: | | | |
| | Zeit in min | %A | %B | Flussrate in mL/min |
| | 0.0 | 95.0 | 5.0 | 1.6 |
| | 2.0 | 10.0 | 90.0 | 1.6 |
| | 5.0 | 10.0 | 90.0 | 1.6 |
| | 5.5 | 90.0 | 10.0 | 1.6 |
| Methode 4: | Säule: | Zorbax Stable Bond C18, 1.8 μM, 3 x 30 mm | | |
| | Fließmittel A: | Wasser / 0.15% Ameisensäure | | |
| | Fließmittel B: | Acetonitril | | |
| | Gradient: | | | |
| | Zeit in min | %A | %B | Flussrate in mL/min |
| | 0.0 | 95.0 | 5.0 | 1.6 |
| | 1.0 | 10.0 | 90.0 | 1.6 |
| | 2.5 | 10.0 | 90.0 | 1.6 |
| | 2.75 | 95.0 | 5.0 | 1.6 |
| Methode 5: | Säule: | 3.5 μM, 4.6 x 50 mm | | |
| | Detektion: | 180 - 820 nm | | |
| | Fließmittel A: | Wasser / 0.1% Trifluoressigsäure | | |

(fortgesetzt)

| | Zeit in min | %A | %B | Flussrate in mL/min |
|---|---|---|---|---|
| | Fließmittel B: | Acetonitril / 0.1% Trifluoressigsäure | | |
| | Temperatur: | 40°C | | |
| | Gradient: | | | |
| | 0.0 | 95.0 | 5.0 | 1:5 |
| | 2.0 | 0.0 | 100.0 | 1.5 |
| | 2.5 | 0.0 | 100.0 | 1.5 |
| | 2.6 | 95.0 | 5.0 | 1.5 |
| Methode 6: | Säule: | Sunfire C18, 3.5 µM, 4.6 x 50 mm | | |
| | Detektion: | 180 - 820 nm | | |
| | Fließmittel A: | Wasser / 0.1% Trifluoressigsäure | | |
| | Fließmittel B: | Acetonitril / 0.1% Trifluoressigsäure | | |
| | Temperatur: | 40°C | | |
| | Gradient: | | | |
| | Zeit in min | %A | %B | Flussrate in mL/min |
| | 0.0 | 95.0 | 5.0 | 1.5 |
| | 2.0 | 0.0 | 100.0 | 1.5 |
| | 3.0 | 0.0 | 100.0 | 1.5 |
| | Zeit in min | %A | %B | Flussrate in mL/min |
| | 3.4 | 95.0 | 5.0 | 1.5 |
| Methode 7: | Säule: | YMC-Pack ODS-AQ, 3 µM, 4.6 x 75 mm | | |
| | Fließmittel A: | Wasser / 0.15% Ameisensäure | | |
| | Fließmittel B: | Acetonitril | | |
| | Gradient: | | | |
| | Zeit in min | %A | %B | Flussrate in mL/min |
| | 0.0 | 95.0 | 5.0 | 1.6 |
| | 4.5 | 10.0 | 90.0 | 1.6 |
| | 5.0 | 10.0 | 90.0 | 1.6 |
| | 5.50 | 90.0 | 10.0 | 1.6 |
| Methode 8: | Säule: | Zorbax Stable Bond C18, 1.8 µM, 3 x 30 mm | | |
| | Fließmittel A: | Wasser / 0.15% Ameisensäure | | |
| | Fließmittel B: | Acetonitril | | |
| | Gradient: | | | |
| | Zeit in min | %A | %B | Flussrate in mL/min |
| | 0.0 | 95.0 | 5.0 | 1.6 |
| | 2.00 | 50.0 | 50.0 | 1.6 |
| | 2.25 | 10.0 | 90.0 | 1.6 |
| | 2.50 | 10.0 | 90.0 | 1.6 |
| | 2.75 | 95.0 | 5.0 | 1.6 |

(fortgesetzt)

| | Zeit in min | %A | %B | Flussrate in mL/min |
|---|---|---|---|---|
| Methode 9: | Säule:<br>Fließmittel A:<br>Fließmittel B: | Zorbax Stable Bond C18, 1.8 μM, 3 x 30 mm<br>Wasser / 0.15% Ameisensäure<br>Acetonitril | | |
| | Gradient: | | | |
| | Zeit in min | %A | %B | Flussrate in mL/min |
| | 0.0 | 95.0 | 5.0 | 1.6 |
| | 2.25 | 10.0 | 90.0 | 1.6 |
| | 2.50 | 10.0 | 90.0 | 1.6 |
| | 2.75 | 95.0 | 5.0 | 1.6 |
| Methode 10: | Säule:<br>Fließmittel A:<br>Fließmittel B:<br>Gradient: | Zorbax Stable Bond C18, 3.5 μM, 4.6 x 75 mm<br>Wasser / 0.15% Ameisensäure<br>Acetonitril | | |
| | Zeit in min | %A | %B | Flussrate in mL/min |
| | 0.0 | 95.0 | 5.0 | 1.6 |
| | 4.5 | 10.0 | 90.0 | 1.6 |
| | 5.0 | 10.0 | 90.0 | 1.6 |
| | 5.50 | 90.0 | 10.0 | 1.6 |
| Methode 11: | Säule:<br>Detektion:<br>Fließmittel A:<br>Fließmittel B:<br>Temperatur:<br>Gradient: | X Terra C18, 3.5 μM, 4.6 x 50 mm<br>180 - 820 nm<br>Wasser / 0.1 % Trifluoressigsäure<br>Acetonitril / 0.1% Trifluoressigsäure<br>40°C | | |
| | Zeit in min | %A | %B | Flussrate in mL/min |
| | 0.0 | 95.0 | 5.0 | 1.5 |
| | 2.0 | 0.0 | 100.0 | 1.5 |
| | 3.0 | 0.0 | 100.0 | 1.5 |
| | 3.4 | 95.0 | 5.0 | 1.5 |
| Methode 12: | Säule:<br>Fließmittel A:<br>Fließmittel B:<br>Gradient: | Merck Cromolith Flash RP18e, 4.6 x 25 mm<br>Wasser / 0.1 % Ameisensäure<br>Acetonitril / 0.1% Ameisensäure | | |
| | Zeit in min | %A | %B | Flussrate in mL/min |
| | 0.0 | 90.0 | 10.0 | 1.6 |
| | 2.7 | 10.0 | 90.0 | 1.6 |
| | 3.0 | 10.0 | 90.0 | 1.6 |
| | 3.3 | 95.0 | 5.0 | 1.6 |
| Methode 13: | Säule:<br>Fließmittel A: | Merck Cromolith SpeedROD RP-18e, 4.6 x 50 mm<br>Wasser / 0.1% Ameisensäure | | |

(fortgesetzt)

| | Zeit in min | %A | %B | Flussrate in mL/min |
|---|---|---|---|---|
| | Fließmittel B: | Acetonitril / 0.1% Ameisensäure | | |
| | Gradient: | | | |
| | 0.0 | 90.0 | 10.0 | 1.5 |
| | 4.5 | 10.0 | 90.0 | 1.5 |
| | 5.0 | 10.0 | 90.0 | 1.5 |
| | 5.5 | 95.0 | 5.0 | 1.5 |
| Methode 14: | Säule: | Zorbax Stable Bond C18, 3.5 µM, 4.6 x 75 mm | | |
| | Fließmittel A: | Wasser / 0.15% Ameisensäure | | |
| | Fließmittel B: | Acetonitril | | |
| | Gradient: | | | |
| | Zeit in min | %A | %B | Flussrate in mL/min |
| | 0.0 | 95.0 | 5.0 | 1.6 |
| | 2.0 | 10.0 | 90.0 | 1.6 |
| | 5.0 | 10.0 | 90.0 | 1.6 |
| | 5.5 | 90.0 | 10.0 | 1.6 |

Folgende präparative Methoden wurden für die Umkehrphasen-Chromatographie verwendet:

| | | | | |
|---|---|---|---|---|
| Methode 1: | Säule: | Atlantis C18, 5 µM, 100 x 30 mm | | |
| | Detektion: | 210 - 500 nm | | |
| | Fließmittel A: | Wasser / 0.1% Trifluoressigsäure | | |
| | Fließmittel B: | Acetonitril | | |
| | Gradient: | | | |
| | Zeit in min | %A | %B | Flussrate in mL/min |
| | 0.0 | 95.0 | 5.0 | 5 |
| | 0.5 | 95.0 | 5.0 | 50 |
| | 8.0 | 5.0 | 95.0 | 50 |
| | 9.0 | 5.0 | 95.0 | 50 |
| | 9.5 | 95.0 | 5.0 | 50 |
| | 10.0 | 95.0 | 5.0 | 50 |
| | 10.1 | 95.0 | 5.0 | 5 |
| Methode 2: | Säule: | Varian Pursuit 5 µM, 50 x 200 mm | | |
| | Fließmittel A: | Wasser / 0.1% Trifluoressigsäure | | |
| | Fließmittel B: | Acetonitril / 0.1% Trifluoressigsäure | | |
| | Gradient: | | | |
| | Zeit in min | %A | %B | Flussrate in mL/min |
| | 0.0 | 95.0 | 5.0 | 180 |
| | 1.15 | 95.0 | 5.0 | 180 |
| | 12.4 | 2.0 | 98.0 | 180 |
| | 14.0 | 2.0 | 98.0 | 180 |

(fortgesetzt)

| | Zeit in min | %A | %B | Flussrate in mL/min |
|---|---|---|---|---|
| | 15.3 | 95.0 | 5.0 | 180 |
| | 15.3 | 95.0 | 5.5 | 180 |
| <u>Methode 3</u>: | Säule:<br>Fließmittel A:<br>Fließmittel B:<br>Gradient: | YMC-Pack ODS-AQ, 5 μM, 30 x 100 mm<br>Wasser / 0.15% Ameisensäure<br>Acetonitril | | |
| | Zeit in min | %A | %B | Flussrate in mL/min |
| | 0.0 | 95.0 | 5.0 | 50 |
| | 2.0 | 95.0 | 5.0 | 50 |
| | 6.0 | 10.0 | 90.0 | 50 |
| | 12.0 | 10.0 | 90.0 | 50 |
| | 13 | 90.0 | 10.0 | 50 |

**Herstellung der Ausgangsverbindungen:**

Die Verbindungen der allgemeinen Formel I können aus den folgenden Intermediaten **A**, **B** und **C** hergestellt werden:

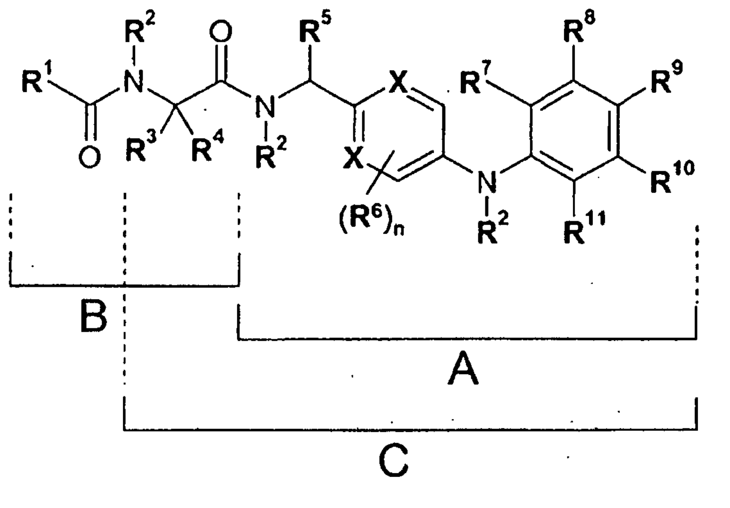

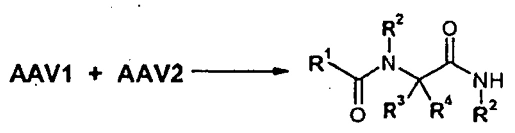


**B**

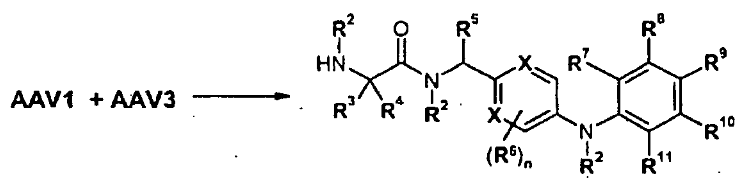


**C**

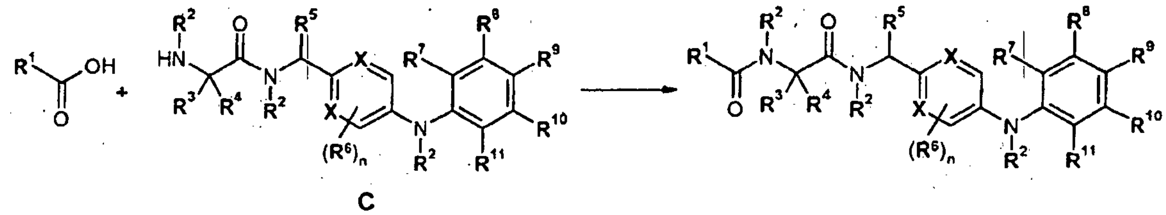

C

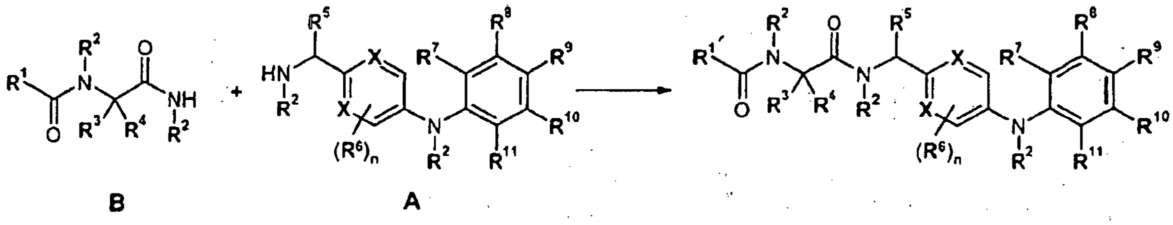

B A

**AAV 1:Amid-Kupplung**

Eine Lösung der Carbonsäure-Komponente (1 Mol-Äquivalent), Triethylamin (2.5 Mol-Äquivatente) und TBTU (1.1 Mol-Äquivalente) in THF wurde 30 Minuten bei Raumtemperatur gerührt. Dann wurde die Amin-Komponente (1.1 Mol-Äquivalent als Hydrochlorid) hinzugegeben und über Nachtweitergerührt. Danach wurde das Gemisch eingedampft, mit Wasser versetzt, mit verdünnter Kaliumcarbonat-Lösung alkalisch gestellt und mit Essigsäureethylester extrahiert. Mittels Säulenchromatographie (entweder Kieselgel oder Reversed Phase Chromatographie) wurde das Produkt isoliert und gereinigt.

**AAV 2**: Ester-Hydrolyse

Zu einer Lösung des Esters (1 Mol-Äquivalent) in Methanol wurde 2N Natronlauge (2 Mol-Äquivalente) gegeben und das Gemisch 1 bis 5 Stunden bei Raumtemperatur gerührt. Danach wurde mit Essigsäure angesäuert und das Gemisch im Vakuum bis zur Trockne eingedampft. Das so erhaltene Rohprodukt wurde üblicherweise durch Säulenchromatographie an Kieselgel gereinigt.

AAV 3:Abspaltung der *tert*-Butyloxycarbonyl-Schutzgruppe

Eine Lösung der *tert*-Butoxycarbonyl-Aminoverbindung (1 Mol-Äquivalent) in Dichlormethan wurde mit Trifluoressigsäure (3 bis 10 Mol-Äquivalente) versetzt und bei Raumtemperatur gerührt, bis die Schutzgruppe vollständig abgespalten war. Das Reaktionsgemisch wurde dann bis zur Trockne eingedampft und das so erhaltene Rohprodukt chromatographisch gereinigt.

**AAV 4**: Herstellung der Intermediate A

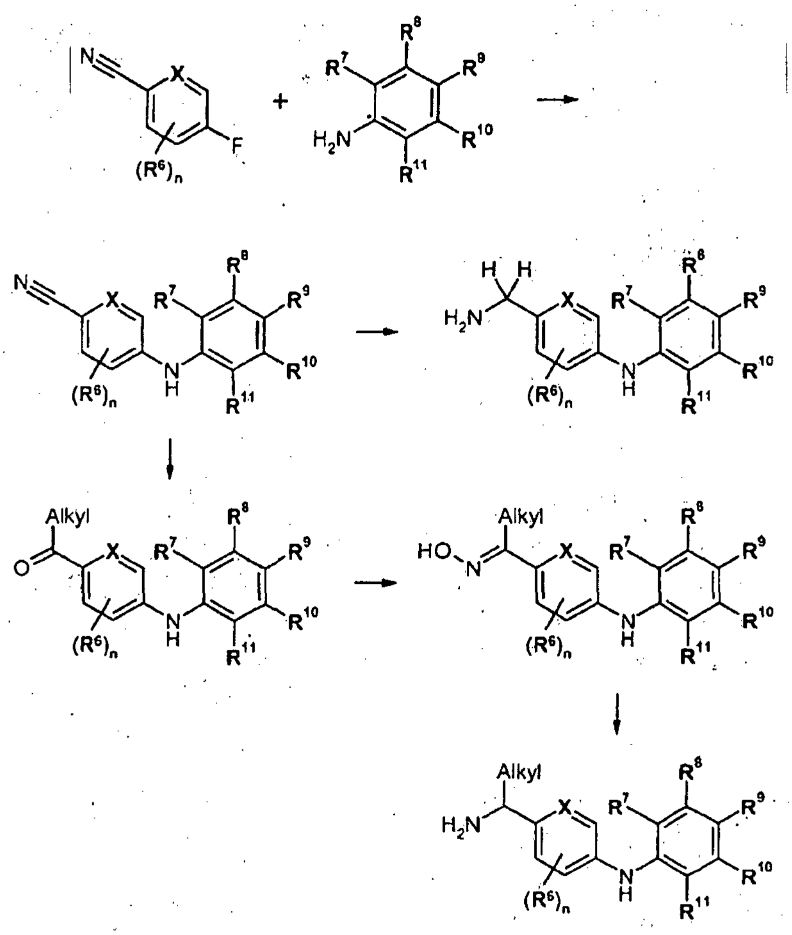

Eine Lösung der Anilin-Komponente (1 Mol-Äquivalent) und einer starken Base wie z.B. Kalium-*tert*-butylat (1 Mol-Äquivalent) in DMSO wurde eine Stunde bei Raumtemperatur gerührt, dann mit der 4-Fluor,benzonitril-Komponente (1 Mol-Äquivalent) versetzt und weiter über Nacht bei ca. 80°C gerührt. Zur Aufarbeitung wurde das Gemisch über Alox filtriert und im Vakuum zur Trockne eingedampft.
Die Nitrilgruppe des so erhaltenen Diphenylamin-Zwischenprodukts wurde anschließend unter Zusatz von Raney-Nickel bei 55°C und 3 bar Wasserstoffüberdruck zur Aminomethylgruppe reduziert und das erhaltene Produkt chromatographisch gereinigt. Zur Herstellung der Intermediate **A** mit alpha-Alkylbenzylgruppe (z.B. A1, A4, A5) wurde das Nitril-Derivat (1 Mol-Äquivalent) in Diethylether gelöst und bei 0 bis 5°C unter Rühren zu einer Lösung von Alkylmagnesiumbromid (4 Mol-Äquivalente) in Diethylether getropft und anschließend noch ca. 30 Minuten nachgerührt. Das Reaktionsgemisch wurde dann bei -5°C in 1 M Salzsäure eingerührt und das so erhaltene Alkylketon auf übliche Weise chromatographisch isoliert und gereingt.
Eine Lösung des so erhaltenen Ketons (1 Mol-Äquivalent) in Acetonitril wurde mit Triethylamin (2 Mol-Äquivalente) und Hydrxylamin-Hydrochlorid (1.3 Mol-Äquivalente) versetzt und 4 Stunden unter Rückfluß erhitzt. Dann wurde Wasser hinzugefügt und mit Dichlormethan extrahiert. Aus der organischen Phase wurde nach üblichem Verfahren das enstandene Oxim isoliert und gereinigt.
Eine Lösung des Oxims (1 Mol-Äquivalent) in Methanol wurde mit methanolischer Salzsäure (6.6 Mol-Äquivalente) versetzt. Nach Zugabe von Zinkstaub (1.4 Mol-Äquivalente) wurde 3 Stunden unter Rühren zum Rückfluß erhitzt. Nach dem Abkühlen wurde das Gemisch mit Wasser versetzt und mit Dichlormethan extrahiert. Wenn notwendig, wurde das so erhaltene Amin chromatographisch gereinigt.
Eine weitere Möglichkeit, das Oxim zum entsprechenden Amin zu reduzieren, besteht in der katalytischen Hydrierung. Dazu wurde das Oxim in methanolischer Ammoniak-Lösung nach Zusatz von Raney-Nickel bei 50°C und einem Wasserstoffüberdruck von 50 psi bis zur vollständigen Wasserstoff-Aufnahme hydriert. Wenn notwendig, wurde das so erhaltene Amin chromatographisch gereinigt.

**Herstellung der Intermediate A**

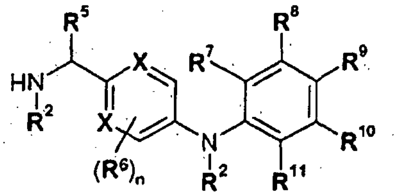

Die folgenden Intermediate A1 bis A9 wurden nach der allgemeinen Arbeitsvorschrift AAV4 hergestellt:

Intermediat A1: (6-Aminomethyl-pyridin-3-yl)-(4-chlor-2-trifluormethyl-phenyl)-amin

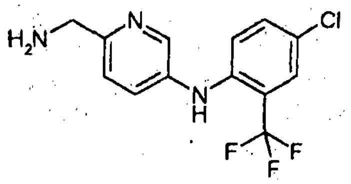

HPLC: $R_t$ = 1.74 Minuten (Methode 13)
Massenspektrum (ESI): [M+H]+ = 302

Intermediat A2: (4-Aminomethyl-phenyl)-(4-fluor-2-trifluormethyl-phenyl)-amin

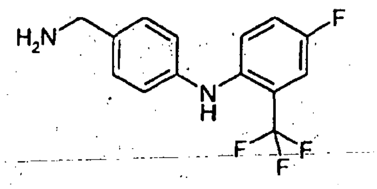

Massenspektrum (ESI): [M+H]+ = 285
Dünnschicht-Chromatogramm (Kieselgel, $CH_2Cl_2$/Ethanol 19:1): $R_f$ = 0.16

Intermediat A3: (6-Aminomethyl-pyridin-3-yl)-(4-fluor-2-trifluormethyl-phenyl)-amin

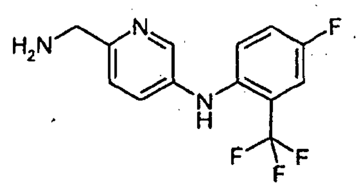

HPLC: $R_t$ = 2.06 Minuten (Methode 3)
Massenspektrum (ESI): [M+H]+ = 286; [M-H]- = 284

Intermediat A4: (4-Aminomethyl-3-fluor-phenyl)-(4-fluor-2-trifluormethyl-phenyl)-amin

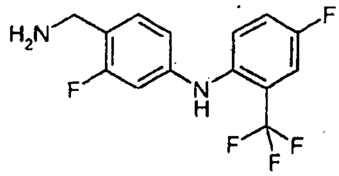

Massenspektrum (ESI): [M+H]+ = 303
Dünnschicht-Chromatogramm (Kieselgel, $CH_2Cl_2$/Ethanol 19:1): $R_f$ = 0.08

Intermediat A5: (4-Aminomethyl-3-fluor-phenyl)-(2-trifluormethyl-phenyl)-amin

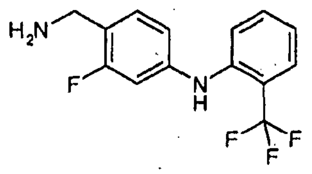

Massenspektrum (ESI): [M-H]- = 283
Dünnschicht-Chromatogramm (Kieselgel, $CH_2Cl_2$/Ethanol 19:1): $R_f$ = 0.09

Intermediat A6: (4-Aminomethyl-phenyl)-(2-trifluormethyl-phenyl)-amin

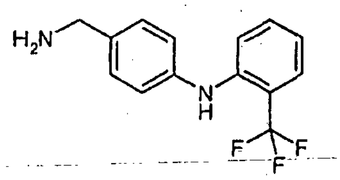

HPLC: $R_t$ = 1.36 Minuten (Methode 1)
Massenspektrum (ESI): $[M+H-NH_3]+$ = 250

Intermediat A7: (4-Aminomethyl-phenyl)-(4-chlor-2-trifluormethyl-phenyl)-amin

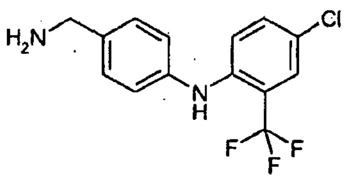

Massenspektrum (ESI): $[M+H-NH_3]+$ = 284/286

Intermediat A8: (4-Aminomethyl-3-fluor-phenyl)-(4-chlor-2-trifluormethyl-phenyl)-amin

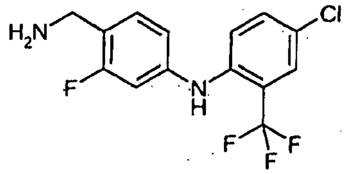

HPLC: $R_t$ = 1.83 Minuten (Methode 2)

Intermediat A9: (4-Aminomethyl-phenyl)-(4-brom-2-trifluormethyl-phenyl)-amin

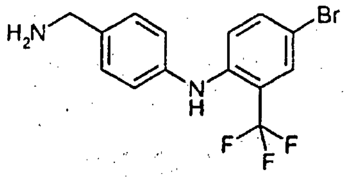

HPLC: $R_t$ = 1.81 Minuten (Methode 2)

**Herstellung der Intermediate B**

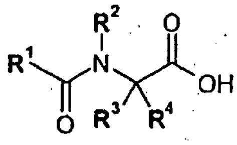

Das folgende Intermediat B1 wurde durch Amid-Kupplung nach der allgemeinen Arbeitsvorschrift AAV1 und anschließende Ester-Verseifung nach der allgemeinen Arbeitsvorschrift AAV2 hergestellt:

Intermediat B1: (S)-3-[(6-Oxo-1,6-dihydro-pyridazin-4-carbonyi)-aminol-tetrahydrofuran-3-carbonsäure

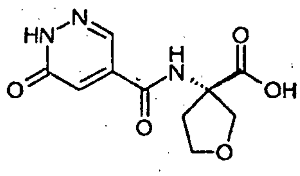

HPLC: $R_t$ = 0.33 Minuten (Methode 2)
Massenspektrum (ESI): [M+H]+ = 254
Analog kann das folgende Intermediat B2 hergestellt werden:

Intermediat B2: 1-[(6-Oxo-1,6-dihydro-pyridazin-4-carbonyl)-amino]-cyclopropancarbonsäure

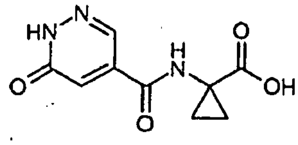

**Herstellung der Intermediate C**

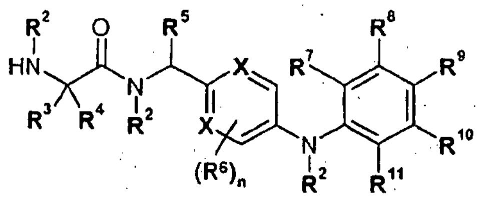

Die folgenden Intermediate C1 bis C6 wurden durch Amid-Kupplung nach der allgemeinen Arbeitsvorschrift AAV1 und anschließender Abspaltung der *tert*-Butyloxy-carbonyl-Schutzgruppe nach der allgemeinen Arbeitsvorschrift AAV3 hergestellt:

Intermediat C1: 1-Amino-cyclopropancarbonsäure-[5-(4-chlor-2-trifluormethylphenylamino)-pyridin-2-ylmethyl]-amid

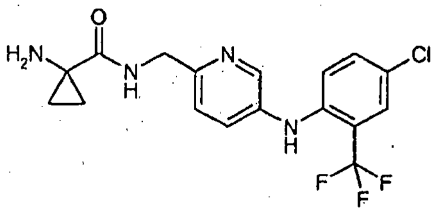

HPLC: $R_t$ = 1.55 Minuten (Methode 13)
Massenspektrum (ESI): [M-H]- = 383

Intermediat C2: 1-Amino-cyclopropancarbonsäure-[5-(4-fluor-2-trifluormethylphenylamino)-pyridin-2-ylmethyl]-amid

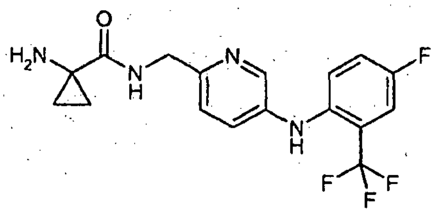

HPLC: $R_t$ = 2.33 Minuten (Methode 7)
Massenspektrum (ESI): [M+H]+ = 369; [M-H]- = 367

Intermediat C3: (*S*)-3-Amino-tetrahydrofuran-3-carbosäure-2-fluor-4-(4-fluor-2-trifluormethyl-phenylamino)-benzylamid

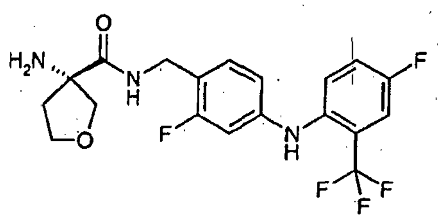

Massenspektrum (ESI): [M+H]+ = 416

Intermediat C4: (*S*)-3-Amino-tetrahydrofuran-3-carbonsäure-4-(4-fluor-2-trifluormethyl-phenylamino)-benzylamid

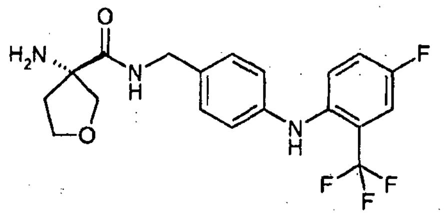

HPLC: $R_t$ = 1.99 Minuten (Methode 2)
Massenspektrum (ESI): [M+H]+ = 398

Intermediat C5: (*S*)-3-Amino-tetrahydrofuran-3-carbonsäure-4-(4-chlor-2-trifluormethyl-phenylamino)-benzylamid

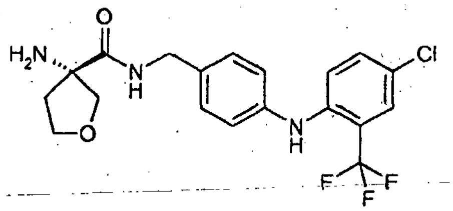

HPLC: $R_t$ = 2.41 Minuten (Methode 2)

Intermediat C6: (*S*)-3-Amino-tetrahydrofuran-3-carbonsäure 2-fluor-4-(2-trifluormethyl-phenylamino)-benzylamid

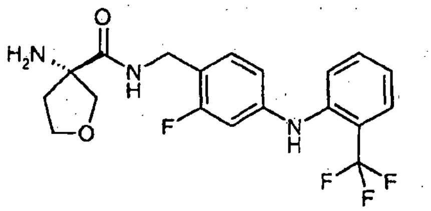

Massenspektrum (ESI): [M+H]+ = 398

**Herstellung der Endverbindungen:**

Beispiel 1: 6-Oxo-5,6-dihydro-pyridazin-4-carbonsäure-(1-{[5-(4-fluor-2-trifluormethylphenylamino)-pyridin-2-ylmethyl]-carbamoyl}-cyclopropyl)-amid

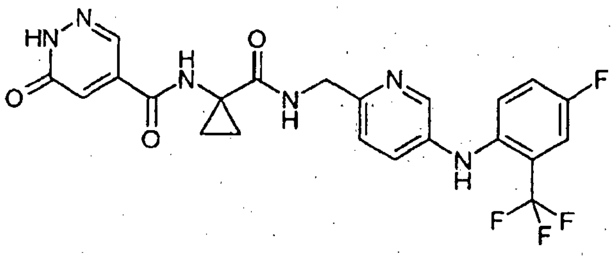

Hergestellt aus Intermediat C2 und 6-Oxo-1,6-dihydro-pyridazin-4-carbonsäure nach AAV1.
$C_{22}H_{18}F_4N_6O_3$ (490.42)
$R_t$ = 2.80 Minuten (Methode 7)

Beispiel 2: 6-Oxo-1,6-dihydro-pyridazin-4-carbonsäure-(1-{[5-(4-chlor-2-trifluormethylphenylamino)-pyridin-2-ylmethyl]-carbamoyl}-cyclopropyl)-amid

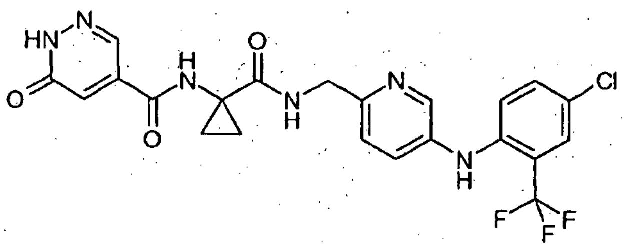

Hergestellt aus Intermediat C1 und 6-Oxo-1,6-dihydro-pyridazin-4-carbonsäure nach AAV1.
$C_{22}H_{18}ClF_3N_6O_3$ (506.87)
$R_t$ = 2.13 Minuten (Methode 2)

Beispiel 3: (*S*)-6-Oxo-1,6-dihydro-pyridazin-4-carbonsäure-{3-[4-(4-fluor-2-trifluormethyl-phenylamiho)-benzylcarbamoyl]-tetrahydrofuran-3-yl}-amid

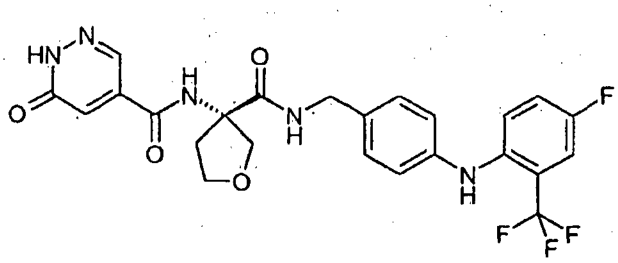

Hergestellt aus Intermédiat C4 und 6-Oxo-1,6-dihydro-pyridazin-4-carbonsäure nach AAV1.
$C_{24}H_{21}F_4N_5O_4$ (519.45)
$R_t$ = 2.39 Minuten (Methode 2)

Beispiel 4: (*S*)-6-Oxo-1,6-dihydro-pyridazin-4-carbonsäure-{3-[4-(4-chlor-2-trifluormethyl-phenylamino)-benzylcarbamoyl]-tetrahydrofuran-3-yl}-amid

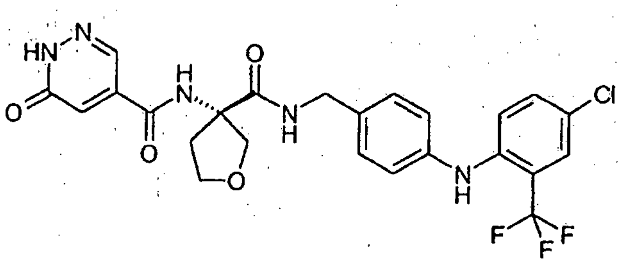

Hergestellt aus den Intermediaten A7 und B1 nach AAV1.
$C_{24}H_{21}ClF_3N_5O_4$ (535.91)
$R_t$ = 2.28 Minuten (Methode 2)

Beispiel 5: (*S*)-6-Oxo-1,6-dihydro-pyridazin-4-carbonsäure-{3-[4-(2-trifluormethylphenylamino)-benzylcarbamoyl]-tetrahydrofuran-3-yl}-amid

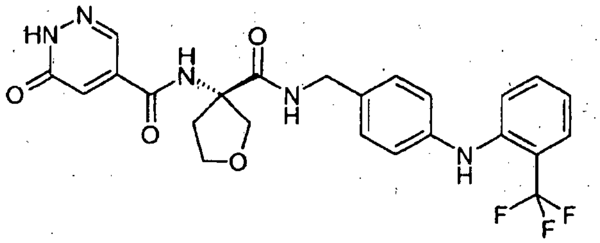

Hergestellt aus den Intermediaten A6 und B1 nach AAV1.
$C_{24}H_{22}F_3N_5O_4$ (501.46)
$R_t$ = 2.09 Minuten (Methode 2)

Beispiel 6: (*S*)-6-Oxo-1,6-dihydro-pyridazin-4-carbonsäure-{3-[2-fluor-4-(4-fluor-2-trifluormethyl-phenylamino)-benzylcarbamoyl]-tetrahydrofuran-3-yl}-amid

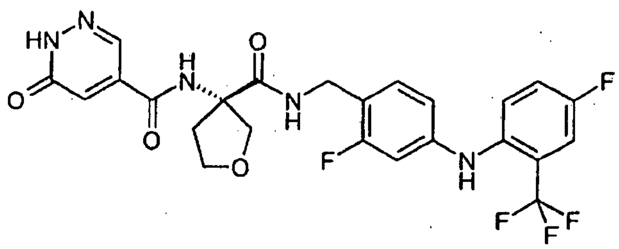

Hergestellt aus den Intermediaten A4 und B1 nach AAV1.
$C_{24}H_{20}F_5N_5O_4$ (537.44)
$R_t$ = 2.15 Minuten (Methode 2)

Beispiel 7: (*S*)-6-Oxo-1,6-dihydro-pyridazin-4-carbonsäure-{3-[4-(4-chlor-2-trifluormethyl-phenylamino)-2-fluor-benzylcarbamoyl]-tetrahydrofuran-3-yl}-amid

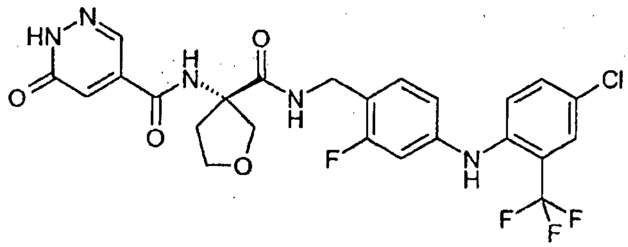

Hergestellt aus den Intermediaten A8 und B1 nach AAV1.
$C_{24}H_{20}ClF_4N_5O_4$ (553.90)
$R_t$ = 2.31 Minuten (Methode 2)

Beispiel 8: (*S*)-6-Oxo-1,6-dihydro-pyridazin-4-carbonsäure-{3-[4-(4-brom-2-trifluormethyl-phenylamino)-benzylcarbamoyl]-tetrahydrofuran-3-yl}-amid

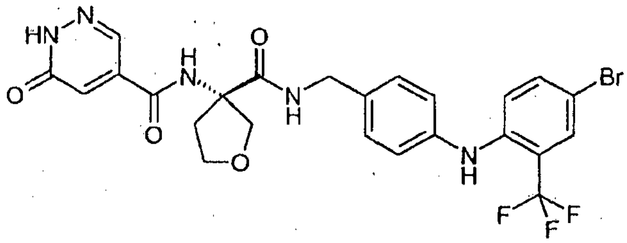

Hergestellt aus den Intermediaten A9 und B1 nach AAV1.
$C_{24}H_{21}BrF_3N_5O_4$ (580.35)
$R_t$ = 2.32 Minuten (Methode 2)

Beispiel.9: (*S*)-6-Oxo-1,6-dihydro-pyridazin-4-carbonsäure {3-[2-fluor-4-(2-trifluormethyl-phenylamino)-benzylcarbamoyl]-tetrahydro-furan-3-yl}-amid

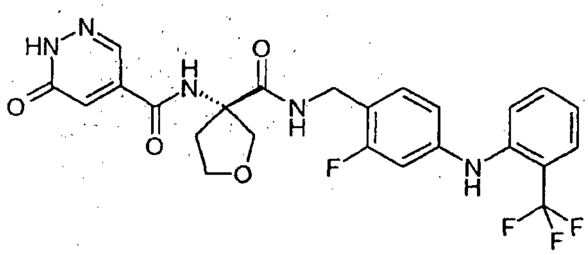

Hergestellt aus den Intermediaten A5 und B1 nach AAV1.

$C_{24}H_{21}F_4N_5O_4$ (519.45)
$R_t$ = 1.35 Minuten (Methode 7)
Massenspektroskopie (ESI): $[M+H]^+$ = 520
$[M-H]^-$ = 518

Die nachfolgenden Beispiele beschreiben pharmazeutische Darreichungsformen, die als Wirkstoff eine beliebige Verbindung der allgemeinen Formel I enthalten, ohne jedoch den Umfang der vorliegenden Erfindung darauf einzuschränken:

Beispiel I

Trockenampulle mit 75 mg Wirkstoff pro 10 ml

Zusammensetzung:

| | |
|---|---|
| Wirkstoff | 75.0 mg |
| Mannitol | 500 mg |
| Wasser für Injektionszwecke ad | 10.0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel II

Tablette mit 50 mg Wirkstoff

Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

Herstellung:

(1), (2) und (3) werden gemischt und mit einerwässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

Beispiel III

Tablette mit 350 mg Wirkstoff

Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |

(fortgesetzt)

| | |
|---|---|
| (5) Magnesiumstearat | 4.0 mg |
| | 600.0 mg |

Herstellung:

(1), (2) und (3) werden gemischt und mit einer wässrigen Lösung von(4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

Beispiel IV

Kapseln mit 50 mg Wirkstoff

Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel V

Kapseln mit 350 mg Wirkstoff

Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Gr6Be 0 abgefüllt.

Beispiel VI

Suppositorien mit 100 mg Wirkstoff

1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitan monostearat | 840.0 mg |
| | 2000.0 mg |

**Patentansprüche**

**1.** Verbindungen der allgemeinen Formel **I**

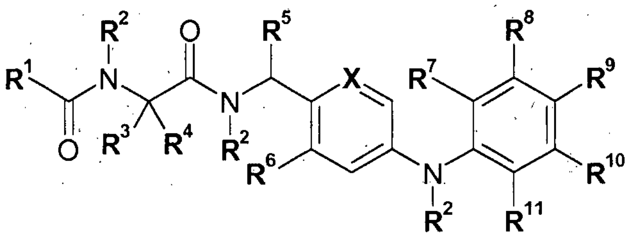

, (**I**)

in der

**$R^1$** die Gruppe

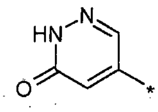

,

**$R^2$** H oder $CH_3$,
**$R^3$** und **$R^4$** zusammen mit dem Kohlenstoffatom, an dem sie gebunden sind, eine $C_{3-6}$-Cycloalkylengruppe, in der eine $-CH_2$-Einheit durch ein Sauerstoffatom ersetzt sein kann,
**$R^5$** H oder $CH_3$,
**$R^6$** H, F, Cl oder Methyl,
**$R^7$** H, F, Cl, Br, -CN, $C_{1-4}$-Alkyl, $CF_3$, $CHF_2$,
**$R^8$** H,
**$R^9$** F, Cl, Br, $C_{1-4}$-Alkyl, -O-$C_{1-4}$-Alkyl, -S-$C_{1-4}$-Alkyl,
**$R^{10}$** H,
**$R^{11}$** F, Cl, Br, -CN, $C_{1-4}$-Alkyl, $CF_3$, $CHF_2$, und
**X** CH oder N bedeuten,

deren Enantiomere, deren Diastereomere, deren Gemische und deren Salze.

**2.** Verbindungen nach Anspruch 1, **dadurch gekennzeichnet, dass**

**$R^2$** H bedeutet,

deren Enantiomere, deren Diastereomere, deren Gemische und deren Salze.

**3.** Verbindungen nach Anspruch 1 oder 2, nämlich:

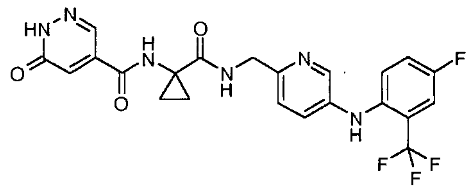

;

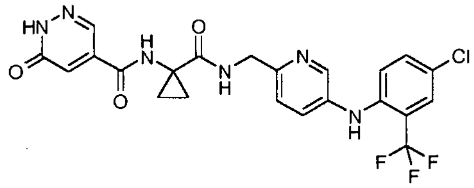

;

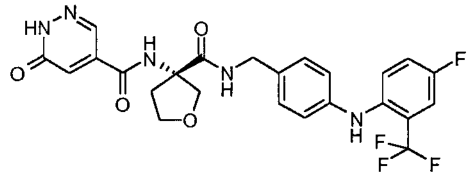

;

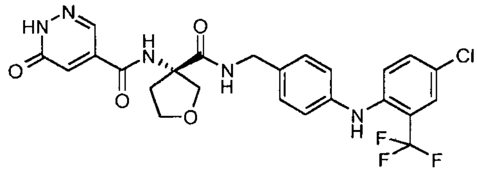

;

;

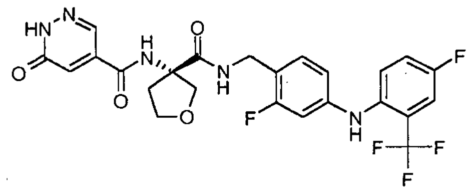

;

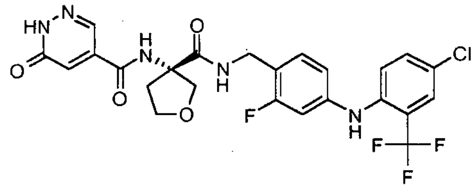

;

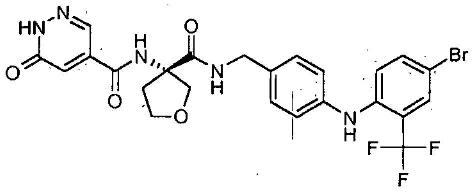

;

deren Enantiomere, deren Diastereomere, deren Gemische und deren Salze.

**4.** Verbindung nach einem der Ansprüche 1 - 3, wobei es sich um die Verbindung

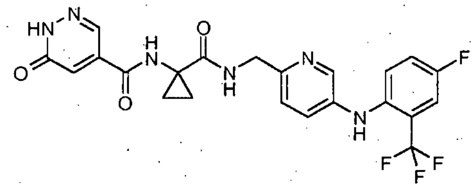

,

deren Enantiomere, deren Diastereomere, deren Gemische oder deren Salze handelt.

**5.** Verbindung nach einem der Ansprüche 1 - 3, wobei es sich um die Verbindung

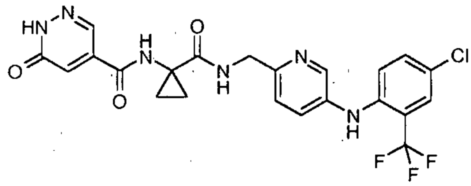

,

deren Enantiomere, deren Diastereomere, deren Gemische oder deren Salze handelt.

**6.** Verbindung nach einem der Ansprüche 1 - 3, wobei es sich um die Verbindung

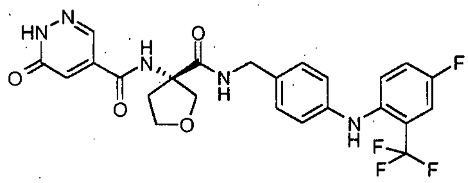

,

deren Enantiomere, deren Diastereomere, deren Gemische oder deren Salze handelt.

**7.** Verbindung nach einem der Ansprüche 1 - 3, wobei es sich um die Verbindung

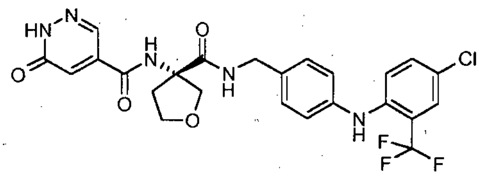

,

deren Enantiomere, deren Diastereomere, deren Gemische oder deren Salze handelt.

**8.** Verbindung nach einem der Ansprüche 1 - 3, wobei es sich um die Verbindung

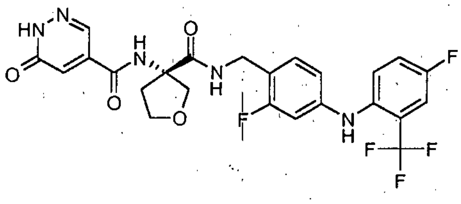

,

deren Enantiomere, deren Diastereomere, deren Gemische oder,deren Salze handelt.

**9.** Verbindung nach einem der Ansprüche 1 - 3, wobei es sich um die Verbindung

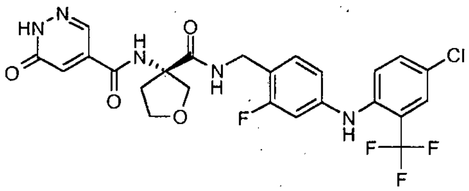

,

deren Enantiomere, deren Diastereomere, deren Gemische oder deren Salze handelt.

**10.** Verbindung nach einem der Ansprüche 1 - 3, wobei es sich um die Verbindung

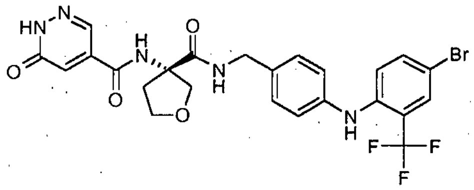

,

deren Enantiomere, deren Diastereomere, deren Gemische oder deren Salze handelt.

**11.** Physiologisch Verträgliche Salze der Verbindungen nach einem der Ansprüche 1-10 mit anorganischen oder organischen Säuren oder Basen.

**12.** Pharmazeutische Zusammensetzung enthaltend eine Verbindung nach einem der Ansprüche 1 - 10 oder ein physiologisch verträgliches Salz nach Anspruch 11, gegebenenfalls mit einem oder mehreren inerten Trägerstoffen und/oder Verdünnungsmitteln.

**13.** Arzneimittel enthaltend eine Verbindung nach einem der Ansprüche 1 - 11.

**14.** Eine Verbindung nach einem der Ansprüche 1 - 11 zur Anwendung in der akuten und prophylaktischen Behandlung von Osteoarthritis, chronisch obstructiver Lungen-Erkrankung, akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen, chronischen Rückenschmerzen und Schmerzen bei diabetischer Neuropathie.

## Claims

**1.** Compounds of general formula I

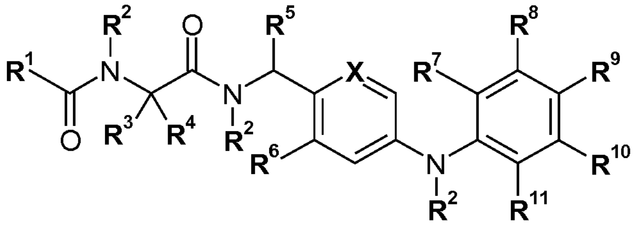

, **(I)**

in which

**$R^1$** denotes the group

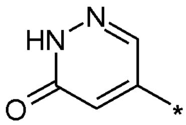,

**$R^2$** denotes H or $CH_3$,
**$R^3$** and **$R^4$** together with the carbon atom to which they are bound denote a $C_{3-6}$-cycloalkylene group in which a $-CH_2$-unit may be replaced by an oxygen atom,
**$R^5$** denotes H or $CH_3$,
**$R^6$** denotes H, F, Cl or methyl,
**$R^7$** denotes H, F, Cl, Br, -CN, $C_{1-4}$-alkyl, $CF_3$, $CHF_2$,
**$R^8$** denotes H,
**$R^9$** denotes F, Cl, Br, $C_{1-4}$-alkyl, -O-$C_{1-4}$-alkyl, -S-$C_{1-4}$-alkyl,
**$R^{10}$** denotes H,
**$R^{11}$** denotes F, Cl, Br, -CN, $C_{1-4}$-alkyl, $CF_3$, $CHF_2$, and
**X** denotes CH or N,

the enantiomers, the diastereomers, the mixtures and the salts thereof.

**2.** Compounds according to claim 1, **characterised in that**

**$R^2$** denotes H,

the enantiomers, the diastereomers, the mixtures and the salts thereof.

**3.** Compounds according to claim 1 or 2, namely:

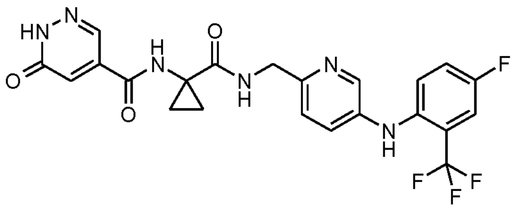;

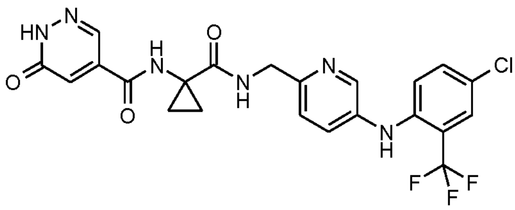;

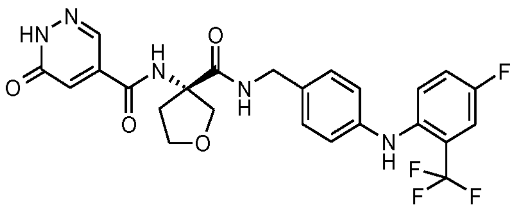;

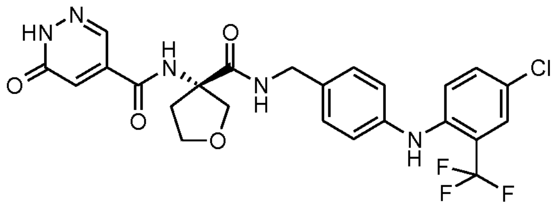

;

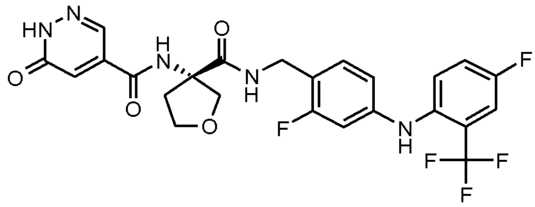

;

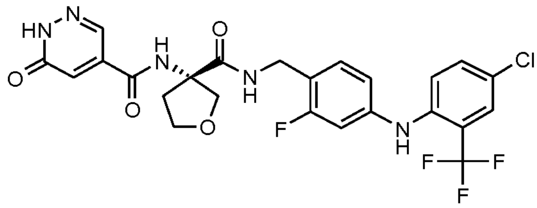

;

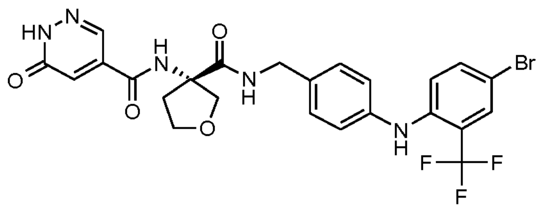

;

the enantiomers, the diastereomers, the mixtures and the salts thereof.

**4.** Compound according to one of claims 1-3, wherein said compound is the compound

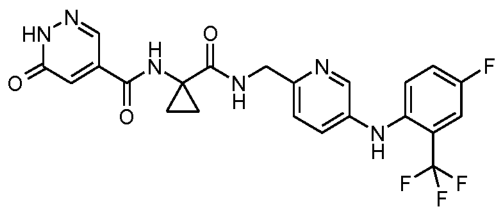

,

the enantiomers, the diastereomers, the mixtures or the salts thereof.

**5.** Compound according to one of claims 1-3, wherein said compound is the compound

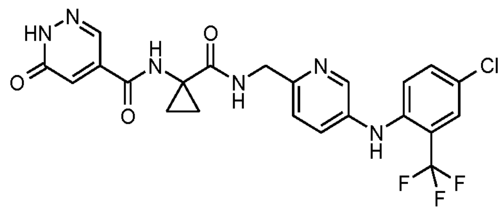

,

the enantiomers, the diastereomers, the mixtures or the salts thereof.

**6.** Compound according to one of claims 1-3, wherein said compound is the compound

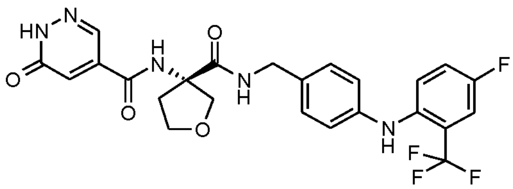

,

the enantiomers, the diastereomers, the mixtures or the salts thereof.

**7.** Compound according to one of claims 1-3, wherein said compound is the compound

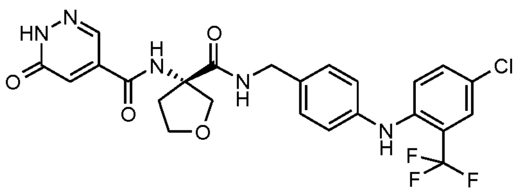

,

the enantiomers, the diastereomers, the mixtures or the salts thereof.

**8.** Compound according to one of claims 1-3, wherein said compound is the compound

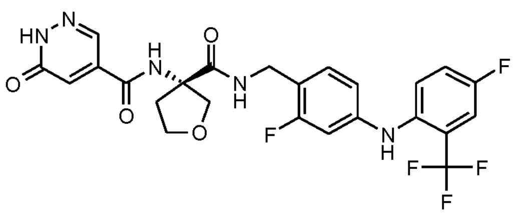

,

the enantiomers, the diastereomers, the mixtures or the salts thereof.

**9.** Compound according to one of claims 1-3, wherein said compound is the compound

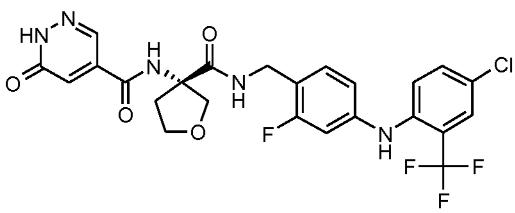

,

the enantiomers, the diastereomers, the mixtures or the salts thereof.

**10.** Compound according to one of claims 1-3, wherein said compound is the compound

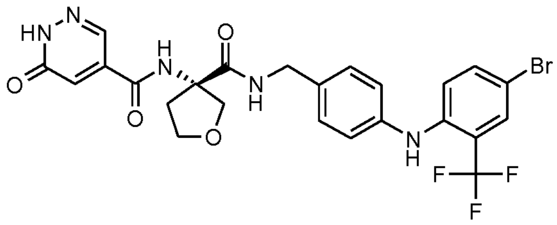

,

the enantiomers, the diastereomers, the mixtures or the salts thereof.

**11.** Physiologically acceptable salts of the compounds according to one of claims 1-10 with inorganic or organic acids or bases.

**12.** Pharmaceutical composition containing a compound according to one of claims 1-10 or a physiologically acceptable salt according to claim 11, optionally with one or more inert carriers and/or diluents.

**13.** Medicaments containing a compound according to one of claims 1-11.

**14.** A compound according to one of claims 1-11, for use in the acute and prophylactic treatment of osteoarthritis, chronic obstructive pulmonary disease, acute pain, visceral pain, neuropathic pain, inflammatory / pain receptor-mediated pain, tumour pain and headache diseases, chronic back pain and pain in diabetic neuropathy.

## Revendications

**1.** Composés de formule générale I

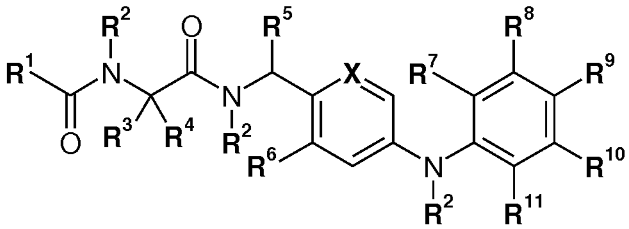

(I)

dans laquelle

$R^1$ représente le groupe

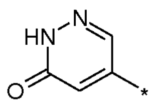

,

$R^2$ est H ou $CH_3$,
$R^3$ et $R^4$ conjointement avec l'atome de carbone auquel ils sont liés représentent un groupe cycloalkylène en $C_3$ à $C_6$, dans lequel un motif $-CH_2-$ peut être remplacé par un atome d'oxygène,
$R^5$ est H ou $CH_3$,
$R^6$ est H, F, Cl ou un groupe méthyle,
$R^7$ est H, F, Cl, Br, -CN, un groupe alkyle en $C_1$ à $C_4$, $CF_3$, $CHF_2$,
$R^8$ est H,
$R^9$ est F, Cl, Br, un groupe alkyle en $C_1$ à $C_4$, -O-alkyle en $C_1$ à $C_4$, -S-alkyle en $C_1$ à $C_4$,

$R^{10}$ est H,
$R^{11}$ est F, Cl, Br, -CN, un groupe alkyle en $C_1$ à $C_4$, $CF_3$, $CHF_2$, et
X est CH ou N,

leurs énantiomères, leurs diastéréomères, leurs mélanges et leurs sels.

**2.** Composés selon la revendication 1, **caractérisés en ce que**

$R^2$ représente H,

leurs énantiomères, leurs diastéréomères, leurs mélanges et leurs sels.

**3.** Composés selon la revendication 1 ou 2, à savoir :

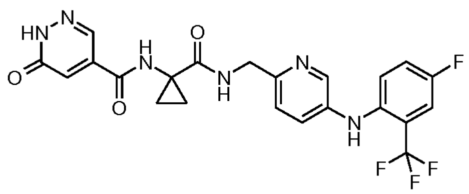

;

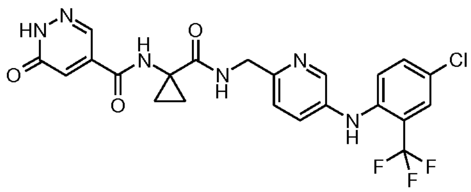

;

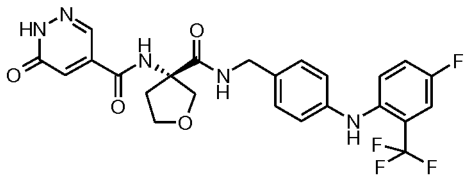

;

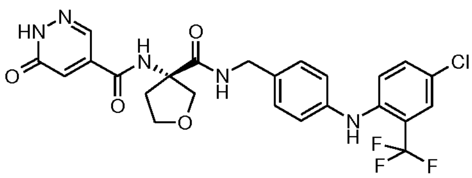

;

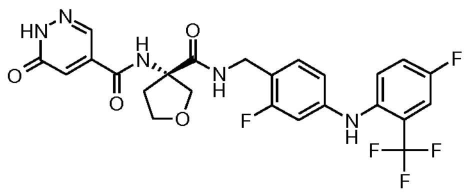

;

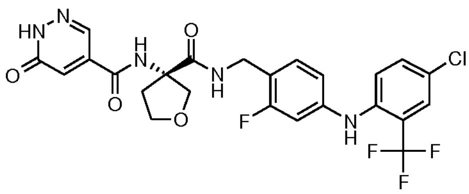

;

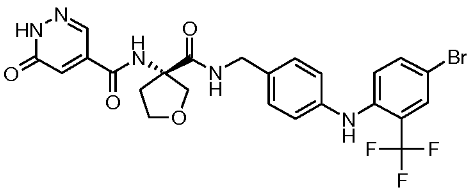

;

leurs énantiomères, leurs diastéréomères, leurs mélanges et leurs sels.

**4.** Composé selon l'une des revendications 1 à 3, dans lequel il s'agit du composé

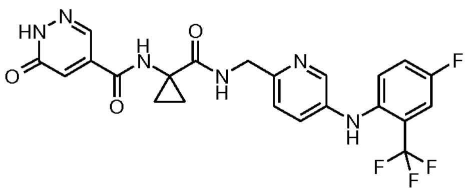

,

de ses énantiomères, de ses diastéréomères, de ses mélanges et de ses sels.

**5.** Composé selon l'une des revendications 1 à 3, dans lequel il s'agit du composé

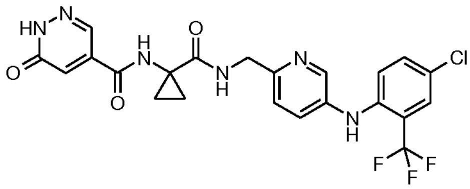

,

de ses énantiomères, de ses diastéréomères, de ses mélanges et de ses sels.

**6.** Composé selon l'une des revendications 1 à 3, dans lequel il s'agit du composé

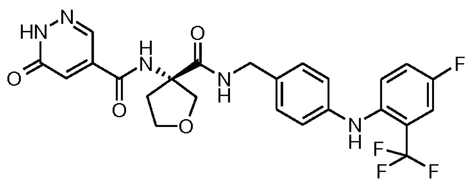

,

de ses énantiomères, de ses diastéréomères, de ses mélanges et de ses sels.

**7.** Composé selon l'une des revendications 1 à 3, dans lequel il s'agit du composé

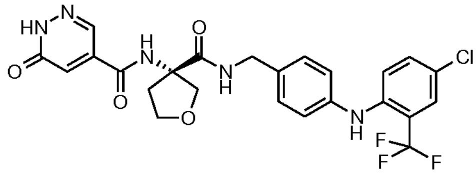

,

de ses énantiomères, de ses diastéréomères, de ses mélanges et de ses sels.

**8.** Composé selon l'une des revendications 1 à 3, dans lequel il s'agit du composé

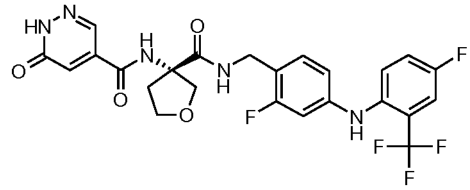

,

de ses énantiomères, de ses diastéréomères, de ses mélanges et de ses sels.

**9.** Composé selon l'une des revendications 1 à 3, dans lequel il s'agit du composé

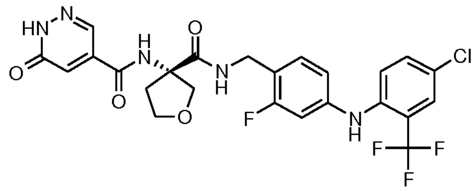

,

de ses énantiomères, de ses diastéréomères, de ses mélanges et de ses sels.

**10.** Composé selon l'une des revendications 1 à 3, dans lequel il s'agit du composé

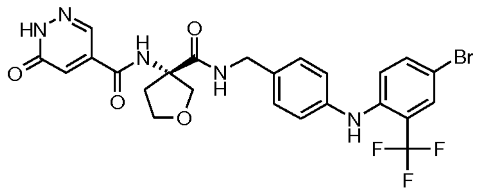

,

de ses énantiomères, de ses diastéréomères, de ses mélanges et de ses sels.

**11.** Sels physiologiquement acceptables des composés selon l'une des revendications 1 à 10, avec des acides ou bases inorganiques ou organique.

**12.** Composition pharmaceutique contenant un composé selon l'une des revendications 1 à 10 ou sel physiologiquement acceptable selon la revendication 11, éventuellement conjointement avec un ou plusieurs véhicules et/ou diluants inertes.

**13.** Médicament contenant un composant selon l'une des revendications 1 à 11.

**14.** Composé selon l'une quelconque des revendications 1 à 11, pour son utilisation dans le traitement aigu et prophylactique de l'arthrose, d'une maladie pulmonaire chronique obstructive, de douleurs aiguës, de douleurs viscérales, de douleurs neuropathiques, de douleurs inflammatoires / induites par un récepteur de la douleur, des douleurs

tumorales et des céphalées, de douleurs dorsales chroniques et de douleurs dans la neuropathie diabétique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2010052232 W
- WO 2005085198 A
- WO 2003066577 A
- WO 2004019868 A
- WO 2005016886 A
- WO 2006120176 A

### In der Beschreibung aufgeführte Nicht-Patentliteratur

- **HOUBEN-WEYL.** *Methoden der Organischen Chemie,* vol. 15/2
- **HOUBEN-WEYL.** *Methoden der Organischen Chemie,* vol. E5/1